# EUROPEAN PATENT APPLICATION

(11) **EP 4 711 385 A1**
(43) Date of publication of application: **18.03.2026**
(21) Application number: 24807397.5
(22) Date of filing: 25.04.2024
(51) Int. Cl.: C07K 16/28, A61P 35/00, A61K 39/00

(54) **DUAL ANTIGEN-TARGETING PROTEIN COMPLEX COMPRISING ANTIBODY OR ANTIGEN-BINDING FRAGMENT THEREOF AND AFFIBODY**

(30) Priority: 12.05.2023 KR 20230062007
(71) Applicant: AbClon Inc., Seoul 08381 (KR)
(72) Inventor: LEE, Jong-Seo, Seongnam-si Gyeonggi-do 13473 (KR); KIM, Jong-Hoon, Gwacheon-si Gyeonggi-do 13835 (KR); KIM, Dong-Wook, Suwon-si Gyeonggi-do 16332 (KR); LEE, Hyun-Jong, Incheon 21348 (KR); AHN, Sang-Ho, Seoul 01746 (KR)
(74) Representative: J A Kemp LLP
(86) International application number: PCT/KR2024/005598
(87) International publication number: WO 2024/237519

(57) **Abstract**

The present disclosure relates to a bispecific antigen-targeting protein complex comprising an antibody or an antigen-binding fragment thereof and an affibody. The bispecific antigen-targeting protein complex of the present disclosure, or a composition comprising the same, can effectively prevent or treat cancer.

## Description

### Technical Field

This patent application claims priority to Korean Patent Application No. 10-2023-0062007, filed on May 12, 2023, with the Korean Intellectual Property Office, the disclosure of which is incorporated herein by reference.

The present invention relates to a dual antigen targeting protein complex comprising an antibody or an antigen-binding fragment thereof, and affibodies.

### Background Art

Monoclonal antibodies (mAbs) have proven to be valuable tools for the treatment of various types of cancer. However, the increasing clinical use of mAbs has highlighted the limitations of monotherapy in cancer treatment. These limitations are particularly evident in cancers with complex mechanisms or those that rapidly acquire resistance or tolerance following monotherapy.

Dual antigen targeting protein complexes, such as bispecific antibodies, are engineered proteins capable of simultaneously binding to two different types of antigens or two distinct epitopes. Such bispecific protein complexes have opened up a wide range of applications, including the redirection of T cells toward tumor cells, dual targeting of different disease mediators, and the delivery of payloads to target sites. For example, the approvals of catumaxomab (anti-EpCAM and anti-CD3) and blinatumomab (anti-CD19 and anti-CD3) marked significant milestones in the research and development of bispecific antibodies.

In light of the limitations of monotherapy, there has been growing interest in therapeutic approaches that combine two drugs acting on different targets to produce a synergistic effect. As drugs acting on dual targets have been shown to exhibit greater therapeutic efficacy than single-agent treatments, there is an urgent need to develop structurally stable dual antigen targeting protein complexes with superior anticancer activity.

### Disclosure of Invention

### Technical Problem

The present inventors have made extensive research efforts to develop a dual antigen targeting protein complex exhibiting superior anticancer activity. As a result, the present inventors have completed the present disclosure by discovering that, when an antibody or an antigen-binding fragment thereof targeting one antigen is combined with an affibody targeting a different antigen, the resulting dual antigen targeting protein complex shows excellent binding affinity to each antigen and superior anticancer efficacy.

Accordingly, an aspect of the present disclosure is to provide a dual antigen targeting protein complex comprising: a first antigen-targeting domain comprising an antibody or an antigen-binding fragment thereof; and a second antigen-targeting domain comprising affibodies bound to the antibody or the antigen-binding fragment thereof.

Another aspect of the present disclosure is to provide a nucleic acid molecule comprising a nucleotide sequence encoding the dual antigen targeting protein complex.

Another aspect of the present disclosure is to provide a recombinant vector comprising the nucleic acid molecule.

Another aspect of the present disclosure is to provide a host cell comprising the recombinant vector.

Another aspect of the present disclosure is to provide a pharmaceutical composition for the prevention or treatment of cancer, comprising a dual antigen targeting protein complex comprising: an EGFR (Epidermal Growth Factor Receptor) or PSMA (Prostate-Specific Membrane Antigen) targeting domain comprising an antibody or an antigen-binding fragment thereof; and a CD137 targeting domain comprising affibodies bound to the antibody or the antigen-binding fragment thereof.

Another aspect of the present disclosure is to provide a method for preventing or treating cancer, comprising administering a dual antigen targeting protein complex comprising: an EGFR (Epidermal Growth Factor Receptor) or PSMA (Prostate-Specific Membrane Antigen) targeting domain comprising an antibody or an antigen-binding fragment thereof; and a CD137 targeting domain comprising affibodies bound to the antibody or the antigen-binding fragment thereof.

Other aspects and advantages of the present disclosure will become more apparent from the following detailed description, claims, and accompanying drawings.

### Solution to Problem

In accordance with an aspect thereof, the present disclosure provides a dual antigen targeting protein complex comprising: a first antigen-targeting domain comprising an antibody or an antigen-binding fragment thereof; and a second antigen-targeting domain comprising an affibodies bound to the antibody or the antigen-binding fragment thereof.

The present inventors have made extensive research efforts to develop a dual antigen targeting protein complex exhibiting superior anticancer activity. As a result, the present inventors have discovered that, when an antibody or an antigen-binding fragment thereof targeting one antigen is combined with an affibody targeting a different antigen, the resulting dual antigen targeting protein complex shows excellent binding affinity to each antigen and superior anticancer efficacy.

As used herein, the term "dual antigen targeting protein complex" may refer to, for example, a bispecific antibody in which two different antigen-binding domains are combined, a fusion protein in which an antibody is combined with an antigen-binding peptide, or a chimeric protein, but is not limited thereto. Preferably, in the present disclosure, the term "dual antigen targeting protein complex" refers to a structure in which an antibody or an antigen-binding fragment thereof is bound to affibodies.

As used herein, the term "dual antigen targeting protein complex" refers to a protein structure capable of simultaneously recognizing and binding to two different antigens.

In accordance with an aspect thereof, the dual antigen targeting protein complex of the present disclosure is a structure in which an antibody or an antigen-binding fragment is linked to an affibody. The antibody or antigen-binding fragment and affibodies of the present disclosure are covalently linked to each other, and, according to one embodiment of the present disclosure, the dual antigen targeting protein complex may be implemented in the form of a fused protein or a conjugate.

Accordingly, the antibody or antigen-binding fragment and the affibodies may be linked by chemical conjugation (as known in the field of organic chemistry) or by other means, such as expressing the complex as a fusion protein, or indirectly via a linker (e.g., an amino acid linker), either directly or indirectly.

According to an embodiment of the present disclosure, the antibody or antigen-binding fragment and the affibodies constituting the protein complex are linked by at least one linker. In this case, the linker may comprise an amino acid sequence represented by the general formula (GnSm)p or (SmGn)p, wherein:
n, m, and p are independently,
n is an integer from 1 to 7;
m is an integer from 0 to 7;
the sum of n and m is an integer of 8 or less; and
p is an integer from 1 to 7.

According to another embodiment of the present disclosure, n is from 1 to 5 and m is from 0 to 5. In a more specific embodiment, n is 4 and m is 1.

In an even more specific embodiment, the linker is GGGGS.

As used herein, the term "antibody" includes not only a full-length antibody but also an antigen-binding fragment of an antibody molecule.

A full-length antibody has a structure comprising two full-length light chains and two full-length heavy chains, each light chain being linked to a heavy chain by a disulfide bond.

The constant region of the heavy chain may be of the γ (gamma), µ (mu), α (alpha), δ (delta), or ε (epsilon) type, and may include subclasses such as γ1, γ2, γ3, γ4, α1, and α2. The constant region of the light chain may be of the kappa (κ) or lambda (λ) type (Cellular and Molecular Immunology, Wonsiewicz, M. J., Ed., Chapter 45, pp. 41-50, W. B. Saunders Co., Philadelphia, PA (1991); Nisonoff, A., Introduction to Molecular Immunology, 2nd Ed., Chapter 4, pp. 45-65, Sinauer Associates, Inc., Sunderland, MA (1984)).

As used herein, the term "antigen-binding fragment" refers to a fragment retaining antigen-binding activity and includes Fab, F(ab'), F(ab')2, chemically linked F(ab')2, and Fv, among others. Among antibody fragments, Fab has a structure comprising the variable region of the light chain, the variable region of the heavy chain, the constant region of the light chain, and the first constant region (CH1) of the heavy chain, and possesses one antigen-binding site. Fab' differs from Fab in that it includes a hinge region having one or more cysteine residues at the C-terminus of the CH1 domain of the heavy chain. An F(ab')2 antibody is generated when the cysteine residues in the hinge regions of two Fab' fragments form disulfide bonds. Fv is the smallest antibody fragment, consisting only of the variable regions of the heavy and light chains. Recombinant techniques for producing Fv fragments are disclosed in PCT International Patent Publications WO 88/10649, WO 88/106630, WO 88/07085, WO 88/07086, and WO 88/09344. A two-chain Fv is a structure in which the variable regions of the heavy and light chains are linked by non-covalent bonds, whereas a single-chain Fv (scFv) is generally a structure in which the variable region of the heavy chain and the variable region of the light chain are covalently linked via a peptide linker or are directly connected at the C-terminus so as to form a dimer-like structure similar to that of a two-chain Fv. Such antibody fragments can be obtained by proteolytic digestion (for example, papain digestion of a full-length antibody yields Fab, while pepsin digestion yields F(ab')2 fragments) or can be prepared using recombinant DNA technology.

In the present disclosure, the antibody is specifically in the form of an scFv or a full-length antibody. In addition, the constant region of the heavy chain may be selected from any one isotype of γ (gamma), µ (mu), α (alpha), δ (delta), or ε (epsilon). The constant region of the light chain may be of the kappa (κ) or lambda (λ) type.

As used herein, the term "heavy chain" refers to both a full-length heavy chain, comprising a variable region domain (VH) including an amino acid sequence having a sufficient variable region sequence to confer antigen specificity, and three constant region domains (CH1, CH2, and CH3), and a fragment thereof. As used herein, the term "light chain" refers to both a full-length light chain, comprising a variable region domain (VL) including an amino acid sequence having a sufficient variable region sequence to confer antigen specificity, and a constant region domain (CL), and a fragment thereof.

As used herein, the term "CDR (complementarity determining region)" refers to the amino acid sequences of the hypervariable regions of the heavy and light chains of immunoglobulins (Kabat et al., Sequences of Proteins of Immunological Interest, 4th Ed., U.S. Department of Health and Human Services, National Institutes of Health (1987)). The heavy chain (CDR-H1, CDR-H2, and CDR-H3) and the light chain (CDR-L1, CDR-L2, and CDR-L3) each comprise three CDRs. The CDRs provide the key contact residues for the binding of an antibody to an antigen or epitope.

As used herein, the antibody or antigen-binding fragment thereof includes not only full-length or native polyclonal or monoclonal antibodies, but also antigen-binding fragments thereof (e.g., Fab, Fab', F(ab')2, Fab3, Fv, and variants thereof), fusion proteins comprising one or more portions of an antibody, human antibodies, humanized antibodies, chimeric antibodies, minibodies, diabodies, triabodies, tetrabodies, linear antibodies, single-chain antibodies (scFv), scFv-Fc, bispecific antibodies, multispecific antibodies, and other modified arrangements of immunoglobulin molecules containing an antigen recognition site with the desired specificity, such as glycosylation variants of antibodies, amino acid sequence variants of antibodies, and covalently modified antibodies. Specific examples of modified antibodies and antigen-binding fragments thereof include nanobodies, AlbudAbs, DARTs (dual affinity re-targeting), BiTEs (bispecific T-cell engagers), TandAbs (tandem diabodies), DAFs (dual acting Fab), two-in-one antibodies, SMIPs (small modular immunopharmaceuticals), FynomAbs (fynomers fused to antibodies), DVD-Igs (dual variable domain immunoglobulins), CovX-bodies (peptide-modified antibodies), DuoBodies, and triomAbs. The foregoing list of antibodies and antigen-binding fragments thereof is not intended to be limiting.

As used herein, the term "framework (FR)" refers to the variable domain residues other than the hypervariable region (HVR) residues. The FR of a variable domain generally consists of four FR domains: FR1, FR2, FR3, and FR4.

Accordingly, the HVR and FR sequences are generally arranged in the following order in the VH (or VL/Vκ):
(a) FRH1 (framework region 1 of the heavy chain) - CDRH1 (complementarity determining region 1 of the heavy chain) - FRH2 - CDRH2 - FRH3 - CDRH3 - FRH4; and
(b) FRL1 (framework region 1 of the light chain) - CDRL1 (complementarity determining region 1 of the light chain) - FRL2 - CDRL2 - FRL3 - CDRL3 - FRL4.

As used herein, the term "variable region" or "variable domain" refers to the domain of the heavy chain or light chain of an antibody that is involved in binding the antibody to an antigen. The variable domains of the heavy chain (VH) and light chain (VL) of native antibodies generally have similar structures, each domain comprising four conserved framework regions (FRs) and three hypervariable regions (HVRs) (Kindt et al., Kuby Immunology, 6th ed., W.H. Freeman and Co., p. 91 (2007)). A single VH or VL domain may be sufficient to confer antigen-binding specificity. In addition, an antibody that binds to a particular antigen can be isolated using a VH or VL domain from an antibody that binds to the antigen by screening a library of complementary VL or VH domains, respectively.

As used herein, the term "specifically binds" or the like means that an antibody or an antigen-binding fragment thereof, or another construct such as an scFv, forms a relatively stable complex with an antigen under physiological conditions. Specific binding can be characterized by an equilibrium dissociation constant (KD) of less than about 1 × 10⁻⁶ M (e.g., 9 × 10⁻⁷ M, 8 × 10⁻⁷ M, 7 × 10⁻⁷ M, 6 × 10⁻⁷ M, 5 × 10⁻⁷ M, 4 × 10⁻⁷ M, 3 × 10⁻⁷ M, 2 × 10⁻⁷ M, or 1 × 10⁻⁷ M), preferably less than 1 × 10⁻⁷ M (e.g., 9 × 10⁻⁸ M, 8 × 10⁻⁸ M, 7 × 10⁻⁸ M, 6 × 10⁻⁸ M, 5 × 10⁻⁸ M, 4 × 10⁻⁸ M, 3 × 10⁻⁸ M, 2 × 10⁻⁸ M, or 1 × 10⁻⁸ M), and more preferably less than 1 × 10⁻⁸ M (e.g., 9 × 10⁻⁹ M, 8 × 10⁻⁹ M, 7 × 10⁻⁹ M, 6 × 10⁻⁹ M, 5 × 10⁻⁹ M, 4 × 10⁻⁹ M, 3 × 10⁻⁹ M, 2 × 10⁻⁹ M, or 1 × 10⁻⁹ M), wherein a smaller KD indicates tighter binding. Methods for determining whether two molecules specifically bind to each other are well known in the art and include, for example, equilibrium dialysis and surface plasmon resonance.

As used herein, the term "affinity" refers to the overall strength of the non-covalent interactions between a single binding site of a molecule (e.g., an antibody) and its binding partner (e.g., an antigen). Unless otherwise specified, the term "binding affinity" as used herein refers to the intrinsic binding affinity reflecting a 1:1 interaction between members of a binding pair (e.g., an antibody and an antigen). The affinity between molecule Y and its partner Y' can generally be expressed as a dissociation constant (Kd). Affinity may be measured by conventional methods known in the art, including those described herein.

As used herein, the term "human antibody" refers to an antibody having an amino acid sequence corresponding to an amino acid sequence of an antibody produced by a human or human cells, or derived from a non-human source utilizing human antibody repertoires or other human antibody-encoding sequences. This definition of a human antibody excludes humanized antibodies that include non-human antigen-binding residues.

As used herein, the term "chimeric antibody" refers to an antibody in which a portion of the heavy chain and/or light chain is derived from a particular source or species, and the remainder of the heavy chain and/or light chain is derived from a different source or species.

As used herein, the term "humanized antibody" refers to a chimeric immunoglobulin, an immunoglobulin chain thereof, or a fragment thereof (e.g., Fv, Fab, Fab', F(ab')2, or another antigen-binding sub-sequence of an antibody) that contains minimal sequence derived from a non-human immunoglobulin (e.g., a mouse immunoglobulin). In most cases, a humanized antibody is a human immunoglobulin (recipient antibody) in which residues of one or more complementarity determining regions (CDRs) have been replaced with residues from CDRs of a non-human species (donor antibody), such as a mouse, rat, or rabbit, having the desired specificity, affinity, and capability. In some cases, framework region (FR) residues of the recipient human immunoglobulin are replaced with corresponding non-human residues. Humanized antibodies may also comprise residues not found in the recipient antibody or in the imported CDR or framework sequences; such modifications are made to further improve and optimize antibody performance. Typically, the humanized antibody will comprise at least one, and generally two, substantially complete variable domains, wherein all or substantially all of the CDR regions correspond to those of a non-human immunoglobulin variable domain, and all or substantially all of the FR regions comprise the sequence of the FR regions of a human immunoglobulin variable domain. The humanized antibody will also comprise at least a portion, and typically all, of the constant region (Fc region) sequence of a human immunoglobulin.

The variants are "substantially similar" in that, when two peptide sequences are optimally aligned, for example, by the GAP or BESTFIT program using default gap weights, they share at least about 90% sequence identity, more preferably at least about 95%, 98%, or 99% sequence identity. Preferably, positions of non-identical residues differ by conservative amino acid substitutions. A "conservative amino acid substitution" refers to a substitution of an amino acid residue with another amino acid residue having a side chain (R group) with similar chemical properties (e.g., charge or hydrophobicity). In general, conservative amino acid substitutions do not substantially alter the functionality of a protein. Where two or more amino acid sequences differ from each other by conservative substitutions, the percentage or degree of similarity may be adjusted upward to account for the conservative nature of the substitutions.

Such amino acid variations are made on the basis of the relative similarity of the amino acid side chain substituents, such as hydrophobicity, hydrophilicity, charge, and size. Analysis of the size, shape, and type of the amino acid side chain substituents shows that arginine, lysine, and histidine are all positively charged residues; alanine, glycine, and serine are of similar size; and phenylalanine, tryptophan, and tyrosine are of similar shape. Therefore, based on these considerations, arginine, lysine, and histidine; alanine, glycine, and serine; and phenylalanine, tryptophan, and tyrosine can be considered biologically functional equivalents.

In introducing variations, the hydropathic index of amino acids may be taken into account. Each amino acid has an assigned hydropathic index according to its hydrophobicity and charge: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The hydrophobic amino acid index is highly important in imparting the interactive biological function of a protein. It is well known that substitution with amino acids having similar hydrophobic indices is required to retain similar biological activity. When introducing variations with reference to the hydrophobic index, substitution is preferably made between amino acids having a hydrophobic index difference of ±2 or less, more preferably ±1 or less, and still more preferably ±0.5 or less.

It is also well known that substitution between amino acids having similar hydrophilicity values results in proteins with equivalent biological activity. As disclosed in U.S. Patent No. 4,554,101, the following hydrophilicity values are assigned to each amino acid residue: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); and tryptophan (-3.4). When introducing variations with reference to hydrophilicity values, substitution is preferably made between amino acids having a hydrophilicity value difference of ±2 or less, more preferably ±1 or less, and still more preferably ±0.5 or less.

Amino acid substitutions in a protein that do not substantially alter the overall activity of the molecule are known in the art (H. Neurath, R. L. Hill, The Proteins, Academic Press, New York, 1979). The most common substitutions occur between amino acid residues such as Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

The antibody or antigen-binding fragment thereof of the present disclosure includes an antibody or antigen-binding fragment thereof comprising minor modifications to the above-described amino acid sequences, i.e., variations that have little or no effect on the three-dimensional structure or function of the antibody. Thus, in some embodiments, even if the sequence does not match exactly with the above-described sequence, it may have at least 100%, 93%, 95%, 96%, 97%, or 98% or more similarity.

As used herein, the term "affibody" is also referred to as "Z body" or "Zb." An affibody is a small protein consisting of 58 amino acid residues. Among these, 13 amino acids in the affibody protein sequence form binding interactions with a target protein, and depending on the amino acid sequence, binding to various target antigens is possible. The sequence can be randomly arranged to construct a library. Similar to antibodies, affibody molecules can be screened from such libraries for binding to various target antigens using methods such as phage display and yeast two-hybrid (Y2H) screening. In addition, affibodies are very small, with a molecular weight of 6.5 kDa, and compared to antibodies in the IgG form, which typically have a molecular weight of 150 kDa, they diffuse systemically upon administration to the human body and are rapidly cleared through renal filtration. Therefore, affibodies are mainly applied in the research and development of diagnostic samples (Goldstein R et al., 2013, Expert Rev Anticancer Ther.)*.*

In accordance with one embodiment of the present disclosure, the dual antigen targeting protein complex comprises an affibody dimer bound to the C-terminus of the heavy chain of the antibody or antigen-binding fragment thereof.

In a particular embodiment of the present disclosure, the dual antigen targeting protein complex is one in which an affibody dimer is bound to the C-terminus of the heavy chain of the antibody or antigen-binding fragment thereof:
the heavy chain of the antibody or antigen-binding fragment thereof consisting of an amino acid sequence set forth in SEQ ID NO: 9, SEQ ID NO: 33, or SEQ ID NO: 57; and
the affibody consisting of an amino acid sequence set forth in SEQ ID NO: 1 or SEQ ID NO: 2.

In the present disclosure, the dimer may be formed by the association of two affibody monomers having the same amino acid sequence. The dimer may be formed by hydrogen bonding, covalent bonding, or other intermolecular forces between each affibody serving as a monomer. Preferably, each affibody constituting the affibody dimer is linked by at least one linker. In this case, the linker may consist of an amino acid sequence represented by the general formula (GnSm)p or (SmGn)p, wherein:
n, m, and p are independently:
n is an integer from 1 to 7;
m is an integer from 0 to 7;
the sum of n and m is an integer of 8 or less; and
p is an integer from 1 to 7.

According to another specific embodiment of the present disclosure, n is from 1 to 5 and m is from 0 to 5. In a more specific embodiment, n is 4 and m is 1.

In an even more specific embodiment, the linker is GGGGS.

In accordance with one embodiment of the present disclosure, the dual antigen targeting protein complex comprises an affibody monomer bound to the C-terminus of each of the heavy chain and the light chain of the antibody or the antigen-binding fragment thereof.

In a particular embodiment of the present disclosure, the dual antigen targeting protein complex comprises an affibody monomer bound to the C-terminus of each of the heavy chain and the light chain of the antibody or the antigen-binding fragment thereof:
the heavy chain of the antibody or antigen-binding fragment thereof consisting of an amino acid sequence set forth in SEQ ID NO: 9, SEQ ID NO: 33, or SEQ ID NO: 57;
the light chain of the antibody or antigen-binding fragment thereof consisting of an amino acid sequence set forth in SEQ ID NO: 10, SEQ ID NO: 34, or SEQ ID NO: 58; and
the affibody consisting of an amino acid sequence set forth in SEQ ID NO: 1 or SEQ ID NO: 2.

In accordance with one embodiment of the present disclosure, the dual antigen targeting protein complex comprises comprises an affibody dimer bound to the C-terminus of the light chain of the antibody or the antigen-binding fragment thereof.

In a particular embodiment of the present disclosure, the dual antigen targeting protein complex comprises comprises an affibody dimer bound to the C-terminus of the light chain of the antibody or the antigen-binding fragment thereof:
the light chain of the antibody or antigen-binding fragment thereof consisting of an amino acid sequence set forth in SEQ ID NO: 10, SEQ ID NO: 34, or SEQ ID NO: 58; and
the affibody consisting of an amino acid sequence set forth in SEQ ID NO: 1 or SEQ ID NO: 2.

In accordance with one embodiment of the present disclosure, the first antigen-targeting domain targets EGFR (Epidermal Growth Factor Receptor) or PSMA (Prostate-Specific Membrane Antigen), and the second antigen-targeting domain targets CD137.

As used herein, the term "EGFR (Epidermal Growth Factor Receptor)" refers to a receptor tyrosine kinase present on the surface of cells. EGFR is primarily involved in regulating important cellular processes such as cell growth, differentiation, and survival. When a ligand such as epidermal growth factor binds to EGFR, intracellular signaling pathways are activated, promoting cell division and growth. EGFR is frequently overexpressed or mutated in various cancers, particularly non-small cell lung cancer, colorectal cancer, and head and neck cancer, and plays an important role in the growth and progression of such cancers.

As used herein, the term "PSMA (Prostate-Specific Membrane Antigen)" refers to a cell membrane protein predominantly expressed in prostate cells. PSMA expression tends to increase in prostate cancer, which indicates its potential use as a target for the diagnosis and treatment of prostate cancer. PSMA also has enzymatic activity and may be involved in nutrient uptake and signal transduction in the extracellular environment.

As used herein, the term "CD137" refers to a co-stimulatory molecule expressed on the surface of immune T cells. CD137 plays an important role in enhancing the activation and survival of T cells. Binding of CD137 to its ligand promotes T cell activation and strengthens immune responses. In cancer therapy, CD137 is being investigated as part of a strategy to enhance the anticancer activity of T cells, particularly in combination with immune checkpoint inhibitors.

In accordance with one embodiment of the present disclosure, the affibody consists of an amino acid sequence set forth in SEQ ID NO: 1 or SEQ ID NO: 2.

The affibody consisting of an amino acid sequence set forth in SEQ ID NO: 1 or SEQ ID NO: 2 represents an affibody targeting CD137.

In accordance with one embodiment of the present disclosure, the antibody or antigen-binding fragment thereof comprises HCDR1, HCDR2, HCDR3, LCDR1, LCDR2, and LCDR3, each consisting of an amino acid sequence set forth in SEQ ID NOs: 3 to 8; HCDR1, HCDR2, HCDR3, LCDR1, LCDR2, and LCDR3, each consisting of an amino acid sequence set forth in SEQ ID NOs: 27 to 32; or HCDR1, HCDR2, HCDR3, LCDR1, LCDR2, and LCDR3, each consisting of an amino acid sequence set forth in SEQ ID NOs: 51 to 56.

In the present disclosure, SEQ ID NOs: 3 to 8 represent the amino acid sequences of HCDR1, HCDR2, HCDR3, LCDR1, LCDR2, and LCDR3, respectively, of an antibody or antigen-binding fragment targeting EGFR. SEQ ID NOs: 27 to 32 represent the amino acid sequences of HCDR1, HCDR2, HCDR3, LCDR1, LCDR2, and LCDR3, respectively, of an antibody or antigen-binding fragment targeting EGFR. SEQ ID NOs: 51 to 56 represent the amino acid sequences of HCDR1, HCDR2, HCDR3, LCDR1, LCDR2, and LCDR3, respectively, of an antibody or antigen-binding fragment targeting PSMA.

In accordance with one embodiment of the present disclosure, the antibody or antigen-binding fragment thereof comprises a heavy chain and a light chain consisting of amino acid sequences set forth in SEQ ID NO: 9 and SEQ ID NO: 10, respectively; a heavy chain and a light chain consisting of amino acid sequences set forth in SEQ ID NO: 33 and SEQ ID NO: 34, respectively; or a heavy chain and a light chain consisting of amino acid sequences set forth in SEQ ID NO: 57 and SEQ ID NO: 58, respectively. In the present disclosure, SEQ ID NO: 9 and SEQ ID NO: 10 represent the amino acid sequences of the heavy chain and light chain, respectively, of an antibody or antigen-binding fragment targeting EGFR. SEQ ID NO: 33 and SEQ ID NO: 34 represent the amino acid sequences of the heavy chain and light chain, respectively, of an antibody or antigen-binding fragment targeting EGFR. SEQ ID NO: 57 and SEQ ID NO: 58 represent the amino acid sequences of the heavy chain and light chain, respectively, of an antibody or antigen-binding fragment targeting PSMA.

In accordance with one embodiment of the present disclosure, the dual antigen targeting protein complex comprises: an affibody monomer-bound heavy chain consisting of an amino acid sequence set forth in SEQ ID NO: 11 and a light chain consisting of an amino acid sequence set forth in SEQ ID NO: 12; an affibody monomer-bound heavy chain consisting of an amino acid sequence set forth in SEQ ID NO: 19 and a light chain consisting of an amino acid sequence set forth in SEQ ID NO: 20; an affibody monomer-bound heavy chain consisting of an amino acid sequence set forth in SEQ ID NO: 35 and a light chain consisting of an amino acid sequence set forth in SEQ ID NO: 36; an affibody monomer-bound heavy chain consisting of an amino acid sequence set forth in SEQ ID NO: 43 and a light chain consisting of an amino acid sequence set forth in SEQ ID NO: 44; an affibody monomer-bound heavy chain consisting of an amino acid sequence set forth in SEQ ID NO: 59 and a light chain consisting of an amino acid sequence set forth in SEQ ID NO: 60; or an affibody monomer-bound heavy chain consisting of an amino acid sequence set forth in SEQ ID NO: 67 and a light chain consisting of an amino acid sequence set forth in SEQ ID NO: 68. In the present disclosure, SEQ ID NOs: 11, 19, 35, and 43 represent sequences of heavy chains of antibodies or antigen-binding fragments targeting EGFR, to which an affibody monomer targeting CD137 is bound. SEQ ID NOs: 59 and 67 represent sequences of heavy chains of antibodies or antigen-binding fragments targeting PSMA, to which an affibody monomer targeting CD137 is bound.

In accordance with one embodiment of the present disclosure, the dual antigen targeting protein complex comprises: an affibody dimer-bound heavy chain consisting of an amino acid sequence set forth in SEQ ID NO: 13 and a light chain consisting of an amino acid sequence set forth in SEQ ID NO: 14; an affibody dimer-bound heavy chain consisting of an amino acid sequence set forth in SEQ ID NO: 21 and a light chain consisting of an amino acid sequence set forth in SEQ ID NO: 22; an affibody dimer-bound heavy chain consisting of an amino acid sequence set forth in SEQ ID NO: 37 and a light chain consisting of an amino acid sequence set forth in SEQ ID NO: 38; an affibody dimer-bound heavy chain consisting of an amino acid sequence set forth in SEQ ID NO: 45 and a light chain consisting of an amino acid sequence set forth in SEQ ID NO: 46; an affibody dimer-bound heavy chain consisting of an amino acid sequence set forth in SEQ ID NO: 61 and a light chain consisting of an amino acid sequence set forth in SEQ ID NO: 62; or an affibody dimer-bound heavy chain consisting of an amino acid sequence set forth in SEQ ID NO: 69 and a light chain consisting of an amino acid sequence set forth in SEQ ID NO: 70. In the present disclosure, SEQ ID NOs: 13, 21, 37, and 45 represent sequences of heavy chains of antibodies or antigen-binding fragments targeting EGFR, bound to affibody dimers targeting CD137. SEQ ID NOs: 61 and 69 represent sequences of heavy chains of antibodies or antigen-binding fragments targeting PSMA, bound to affibody dimers targeting CD137.

In accordance with one embodiment of the present disclosure, the dual antigen targeting protein complex comprises: an affibody monomer-bound heavy chain consisting of an amino acid sequence set forth in SEQ ID NO: 15 and an affibody monomer-bound light chain consisting of an amino acid sequence set forth in SEQ ID NO: 16; an affibody monomer-bound heavy chain consisting of an amino acid sequence set forth in SEQ ID NO: 23 and an affibody monomer-bound light chain consisting of an amino acid sequence set forth in SEQ ID NO: 24; an affibody monomer-bound heavy chain consisting of an amino acid sequence set forth in SEQ ID NO: 39 and an affibody monomer-bound light chain consisting of an amino acid sequence set forth in SEQ ID NO: 40; an affibody monomer-bound heavy chain consisting of an amino acid sequence set forth in SEQ ID NO: 47 and an affibody monomer-bound light chain consisting of an amino acid sequence set forth in SEQ ID NO: 48; an affibody monomer-bound heavy chain consisting of an amino acid sequence set forth in SEQ ID NO: 63 and an affibody monomer-bound light chain consisting of an amino acid sequence set forth in SEQ ID NO: 64; or an affibody monomer-bound heavy chain consisting of an amino acid sequence set forth in SEQ ID NO: 71 and an affibody monomer-bound light chain consisting of an amino acid sequence set forth in SEQ ID NO: 72. In the present disclosure, SEQ ID NOs: 15, 23, 39, and 47 represent sequences of heavy chains of antibodies or antigen-binding fragments targeting EGFR, bound to affibody monomers targeting CD137. SEQ ID NOs: 63 and 71 represent sequences of heavy chains of antibodies or antigen-binding fragments targeting PSMA, bound to affibody monomers targeting CD137.

In the present disclosure, SEQ ID NOs: 16, 24, 40, and 48 represent sequences of light chains of antibodies or antigen-binding fragments targeting EGFR, bound to affibody monomers targeting CD137. SEQ ID NOs: 64 and 72 represent sequences of light chains of antibodies or antigen-binding fragments targeting PSMA, bound to affibody monomers targeting CD137.

In accordance with one embodiment of the present disclosure, the dual antigen targeting protein complex comprises: a heavy chain consisting of an amino acid sequence set forth in SEQ ID NO: 17 and an affibody dimer-bound light chain consisting of an amino acid sequence set forth in SEQ ID NO: 18; a heavy chain consisting of an amino acid sequence set forth in SEQ ID NO: 25 and an affibody dimer-bound light chain consisting of an amino acid sequence set forth in SEQ ID NO: 26; a heavy chain consisting of an amino acid sequence set forth in SEQ ID NO: 41 and an affibody dimer-bound light chain consisting of an amino acid sequence set forth in SEQ ID NO: 42; a heavy chain consisting of an amino acid sequence set forth in SEQ ID NO: 49 and an affibody dimer-bound light chain consisting of an amino acid sequence set forth in SEQ ID NO: 50; a heavy chain consisting of an amino acid sequence set forth in SEQ ID NO: 65 and an affibody dimer-bound light chain consisting of an amino acid sequence set forth in SEQ ID NO: 66; or a heavy chain consisting of an amino acid sequence set forth in SEQ ID NO: 73 and an affibody dimer-bound light chain consisting of an amino acid sequence set forth in SEQ ID NO: 74. In the present disclosure, SEQ ID NOs: 18, 26, 42, and 50 represent sequences of light chains of antibodies or antigen-binding fragments targeting EGFR, bound to affibody dimers targeting CD137. SEQ ID NOs: 66 and 74 represent sequences of light chains of antibodies or antigen-binding fragments targeting PSMA, bound to affibody dimers targeting CD137.

In accordance with one aspect of the present disclosure, the present disclosure provides a nucleic acid molecule comprising a nucleotide sequence encoding the dual antigen targeting protein complex.

As used herein, the term "nucleic acid molecule" broadly refers to DNA (gDNA and cDNA) and RNA molecules, and the nucleotide, which is the basic structural unit of the nucleic acid molecule, includes not only natural nucleotides but also analogues in which the sugar or base portion is modified (Scheit, Nucleotide Analogs, John Wiley, New York (1980); Uhlman and Peyman, Chemical Reviews, 90:543-584 (1990)).

It will be apparent to those skilled in the art that the nucleotide sequence encoding the antibody or antigen-binding fragment thereof of the present disclosure is merely required to be a nucleotide sequence encoding the amino acid sequence constituting the antibody or antigen-binding fragment thereof, and is not limited to any particular nucleotide sequence.

This is because even if a variation occurs in the nucleotide sequence, expression of the variant nucleotide sequence as a protein may not result in a change in the protein sequence. This is referred to as codon degeneracy. Accordingly, the nucleotide sequence includes a nucleotide sequence comprising functionally equivalent codons, codons encoding the same amino acid (for example, due to codon degeneracy, there are six codons for arginine and for serine), or codons encoding biologically equivalent amino acids.

Considering the variations having the above-described biologically equivalent activity, the nucleic acid molecule encoding the amino acid sequence constituting the antibody or antigen-binding fragment thereof is to be construed as also encompassing sequences having substantial identity thereto. The term "substantial identity" refers to a sequence that, when aligned with any other sequence to maximize correspondence and analyzed using an algorithm conventionally employed in the art, exhibits at least 60% homology (e.g., 61%, 62%, 63%, 64%, 65%, 66%, 67%, 68%, or 69%), more specifically at least 70% homology (e.g., 71%, 72%, 73%, 74%, 75%, 76%, 77%, 78%, or 79%), still more specifically at least 80% homology (e.g., 81%, 82%, 83%, 84%, 85%, 86%, 87%, 88%, or 89%), even more specifically at least 90% homology (e.g., 91%, 92%, 93%, 94%, 95%, 96%, 97%, 98%, or 99%), and most specifically at least 95% homology (e.g., 95%, 96%, 97%, 98%, or 99%) with the sequence of the present disclosure described above. All integers from 60% to 100% and all decimal fractions therebetween are encompassed within the scope of the present disclosure with respect to percent homology.

Methods for sequence alignment are well known in the art. Various methods and algorithms for alignment are disclosed in Smith and Waterman, Adv. Appl. Math. 2:482 (1981); Needleman and Wunsch, J. Mol. Biol. 48:443 (1970); Pearson and Lipman, Methods in Mol. Biol. 24:307-31 (1988); Higgins and Sharp, Gene 73:237-44 (1988); Higgins and Sharp, CABIOS 5:151-3 (1989); Corpet et al., Nuc. Acids Res. 16:10881-90 (1988); Huang et al., Comp. Appl. BioSci. 8:155-65 (1992); and Pearson et al., Meth. Mol. Biol. 24:307-31 (1994). The NCBI Basic Local Alignment Search Tool (BLAST) (Altschul et al., J. Mol. Biol. 215:403-10 (1990)) is accessible, for example, from the National Center for Biological Information (NCBI), and is available in connection with sequence analysis programs such as blastp, blastn, blastx, tblastn, and tblastx on the Internet. BLAST can be accessed via the BLAST page on the NCBI website. A method for comparing sequence homology using this program can be found on the BLAST help page of the NCBI website.

In accordance with one embodiment of the present disclosure, the nucleic acid molecule may comprise one or more of the amino acid sequences of SEQ ID NOs: 75 to 148.

SEQ ID NO: 75 and SEQ ID NO: 76 represent nucleotide sequences encoding affibodies targeting CD137.

In the present disclosure, SEQ ID NOs: 77 to 82 represent nucleotide sequences encoding HCDR1, HCDR2, HCDR3, LCDR1, LCDR2, and LCDR3, respectively, of an antibody or antigen-binding fragment targeting EGFR. SEQ ID NOs: 101 to 106 represent nucleotide sequences encoding HCDR1, HCDR2, HCDR3, LCDR1, LCDR2, and LCDR3, respectively, of an antibody or antigen-binding fragment targeting EGFR. SEQ ID NOs: 125 to 130 represent nucleotide sequences encoding HCDR1, HCDR2, HCDR3, LCDR1, LCDR2, and LCDR3, respectively, of an antibody or antigen-binding fragment targeting PSMA.

In the present disclosure, SEQ ID NOs: 83 and 84 represent nucleotide sequences encoding the heavy chain and the light chain, respectively, of an antibody or antigen-binding fragment targeting EGFR. SEQ ID NOs: 107 and 108 represent nucleotide sequences encoding the heavy chain and the light chain, respectively, of an antibody or antigen-binding fragment targeting EGFR. SEQ ID NOs: 131 and 132 represent nucleotide sequences encoding the heavy chain and the light chain, respectively, of an antibody or antigen-binding fragment targeting PSMA.

In the present disclosure, SEQ ID NOs: 85, 93, 109, and 117 represent nucleotide sequences encoding heavy chains of antibodies or antigen-binding fragments targeting EGFR, to which affibody monomers targeting CD137 are bound. SEQ ID NOs: 86, 94, 110, and 118 represent nucleotide sequences encoding the light chains of antibodies or antigen-binding fragments targeting EGFR. SEQ ID NOs: 133 and 141 represent nucleotide sequences encoding heavy chains of antibodies or antigen-binding fragments targeting PSMA, to which affibody monomers targeting CD137 are bound. SEQ ID NOs: 134 and 142 represent nucleotide sequences encoding the light chains of antibodies or antigen-binding fragments targeting PSMA.

In the present disclosure, SEQ ID NOs: 87, 95, 111, and 119 represent nucleotide sequences encoding heavy chains of antibodies or antigen-binding fragments targeting EGFR, bound to affibody dimers targeting CD137. SEQ ID NOs: 88, 96, 112, and 120 represent nucleotide sequences encoding the light chains of antibodies or antigen-binding fragments targeting EGFR. SEQ ID NOs: 135 and 143 represent nucleotide sequences encoding heavy chains of antibodies or antigen-binding fragments targeting PSMA, bound to affibody dimers targeting CD137. SEQ ID NOs: 136 and 144 represent nucleotide sequences encoding the light chains of antibodies or antigen-binding fragments targeting PSMA.

In the present disclosure, SEQ ID NOs: 89, 97, 113, and 121 represent nucleotide sequences encoding heavy chains of antibodies or antigen-binding fragments targeting EGFR, bound to affibody monomers targeting CD137. SEQ ID NOs: 137 and 145 represent nucleotide sequences encoding heavy chains of antibodies or antigen-binding fragments targeting PSMA, bound to affibody monomers targeting CD137.

In the present disclosure, SEQ ID NOs: 90, 98, 114, and 122 represent nucleotide sequences encoding light chains of antibodies or antigen-binding fragments targeting EGFR, bound to affibody monomers targeting CD137. SEQ ID NOs: 138 and 146 represent nucleotide sequences encoding light chains of antibodies or antigen-binding fragments targeting PSMA, bound to affibody monomers targeting CD137.

In the present disclosure, SEQ ID NOs: 91, 99, 115, and 123 represent nucleotide sequences encoding the heavy chains of antibodies or antigen-binding fragments targeting EGFR. SEQ ID NOs: 92, 100, 116, and 124 represent nucleotide sequences encoding the light chains of antibodies or antigen-binding fragments targeting EGFR, bound to affibody dimers targeting CD137. SEQ ID NOs: 139 and 147 represent nucleotide sequences encoding the light chains of antibodies or antigen-binding fragments targeting PSMA. SEQ ID NOs: 140 and 148 represent nucleotide sequences encoding the light chains of antibodies or antigen-binding fragments targeting PSMA, bound to affibody dimers targeting CD137.

In accordance with one aspect of the present disclosure, the present disclosure provides a recombinant vector comprising the nucleic acid molecule.

As used herein, the term "vector" refers to a means for expressing a target gene in a host cell, including but not limited to plasmid vectors; cosmid vectors; and viral vectors such as bacteriophage vectors, adenovirus vectors, retrovirus vectors, and adeno-associated virus vectors.

In accordance with one embodiment of the present disclosure, in the vector of the present disclosure, the nucleic acid molecule is operatively linked to a promoter.

As used herein, the term "operatively linked" refers to a functional linkage between a nucleic acid expression control sequence (e.g., a promoter, signal sequence, or an array of transcription factor binding sites) and another nucleic acid sequence, whereby the control sequence regulates transcription and/or translation of the other nucleic acid sequence.

The recombinant vector system of the present disclosure may be constructed by various methods known in the art, and specific methods therefor are disclosed in Sambrook et al., Molecular Cloning, A Laboratory Manual, Cold Spring Harbor Laboratory Press (2001), which is incorporated herein by reference.

The vector of the present disclosure may typically be constructed as a cloning vector or as an expression vector. In addition, the vector of the present disclosure may be constructed using a prokaryotic cell or a eukaryotic cell as a host.

For example, when the vector of the present disclosure is an expression vector and a eukaryotic cell is used as a host, a promoter derived from the genome of a mammalian cell (e.g., metallothionein promoter, β-actin promoter, human hemoglobin promoter, and human muscle creatine promoter) or a promoter derived from a mammalian virus (e.g., adenovirus late promoter, vaccinia virus 7.5K promoter, SV40 promoter, cytomegalovirus promoter, thymidine kinase (tk) promoter of HSV, mouse mammary tumor virus (MMTV) promoter, LTR promoter of HIV, Moloney virus promoter, Epstein-Barr virus (EBV) promoter, and Rous sarcoma virus (RSV) promoter) may be used, and generally includes a polyadenylation sequence as a transcription termination sequence.

The vector of the present disclosure may be fused to another sequence to facilitate purification of the antibody expressed therefrom. Examples of such fusion sequences include glutathione S-transferase (Pharmacia, USA), maltose-binding protein (NEB, USA), FLAG (IBI, USA), and 6×His (hexahistidine; Qiagen, USA).

Meanwhile, the expression vector of the present disclosure comprises, as a selectable marker, an antibiotic resistance gene commonly used in the art, for example, resistance genes for ampicillin, gentamicin, carbenicillin, chloramphenicol, streptomycin, kanamycin, geneticin, neomycin, and tetracycline.

Optionally, the vector may additionally carry a gene encoding a reporter molecule (e.g., luciferase or β-glucuronidase).

In accordance with one embodiment of the present disclosure, the expression vector is a recombinant vector for host cell expression, in which a nucleic acid molecule encoding the protein complex comprising an antibody or an antigen-binding fragment thereof and affibodies is inserted, the nucleotide sequence of the nucleic acid molecule being operatively linked to a promoter that generates an RNA molecule in the host cell, and comprising a poly(A) signal sequence that functions in the host cell to cause polyadenylation at the 3' end of the RNA molecule.

In accordance with one aspect of the present disclosure, the present disclosure provides a host cell comprising the recombinant vector.

Any host cell known in the art that is capable of stably and continuously cloning and expressing the vector of the present disclosure may be used. For example, suitable eukaryotic host cells for the vector include yeast (*Saccharomyces cerevisiae*), insect cells, monkey kidney cells (COS7), NS0 cells, SP2/0 cells, Chinese hamster ovary (CHO) cells, W138 cells, baby hamster kidney (BHK) cells, MDCK cells, myeloma cell lines, HuT 78 cells, and HEK-293 cells, without limitation.

As used herein, the terms "transformed," "transfected," or "transduced" refer to the process by which exogenous nucleic acid is delivered or introduced into a host cell. A "transformed," "transfected," or "transduced" cell refers to a cell that has been transformed, transfected, or transduced with exogenous nucleic acid, and includes both the cell itself and progeny cells resulting from replication or subculture of the cell.

Methods for introducing the vector of the present disclosure into a host cell, when the host cell is a eukaryotic cell, include, for example, microinjection (Capecchi, M.R., Cell, 22:479 (1980)), calcium phosphate precipitation (Graham, F.L. et al., Virology, 52:456 (1973)), electroporation (Neumann, E. et al., EMBO J., 1:841 (1982)), liposome-mediated transfection (Wong, T.K. et al., Gene, 10:87 (1980)), DEAE-dextran treatment (Gopal, Mol. Cell Biol., 5:1188-1190 (1985)), and gene bombardment (Yang et al., Proc. Natl. Acad. Sci., 87:9568-9572 (1990)).

In the present disclosure, the recombinant vector introduced into the host cell may express the recombinant protein complex within the host cell, thereby obtaining a large amount of the protein complex. For example, when the expression vector comprises a lac promoter, gene expression may be induced in the host cell by treatment with IPTG.

The culture is typically carried out under aerobic conditions, such as shaking culture or rotation in a rotary incubator. The culture temperature is preferably in the range of 10°C to 40°C, and the culture period is generally from 5 hours to 7 days. The pH of the medium is preferably maintained within the range of 3.0 to 9.0 during culture. The pH of the medium may be adjusted using inorganic or organic acids, alkali solutions, urea, calcium carbonate, ammonia, or the like. During culture, antibiotics such as ampicillin, streptomycin, chloramphenicol, kanamycin, and tetracycline may be added as necessary for the maintenance and expression of the recombinant vector. When culturing a host cell transformed with a recombinant expression vector having an inducible promoter, an appropriate inducer may be added to the medium as needed. For example, when the expression vector comprises a lac promoter, IPTG (isopropyl-β-D-thiogalactopyranoside) may be added, and when it comprises a trp promoter, indoleacrylic acid may be added to the medium.

In accordance with one aspect of the present disclosure, the present disclosure provides a pharmaceutical composition for the prevention or treatment of cancer, the composition comprising: a dual antigen targeting protein complex comprising (i) an EGFR (epidermal growth factor receptor) or PSMA (prostate-specific membrane antigen) targeting domain comprising an antibody or an antigen-binding fragment thereof; and (ii) a CD137 targeting domain comprising affibodies bound to the antibody or antigen-binding fragment thereof.

In one embodiment of the present disclosure, the cancer is selected from the group consisting of breast cancer, ovarian cancer, gastric cancer, lung cancer, non-small cell lung cancer, liver cancer, hepatocellular carcinoma, bronchial cancer, nasopharyngeal cancer, laryngeal cancer, pancreatic cancer, bladder cancer, colorectal cancer, colon cancer, rectal cancer, cervical cancer, brain cancer, prostate cancer, bone cancer, head and neck cancer, skin cancer, thyroid cancer, parathyroid cancer, squamous cell carcinoma, peritoneal cancer, kidney cancer, testicular cancer, esophageal cancer, and ureteral cancer; however, it is not limited thereto, and the cancer may be, for example, a carcinoma, lymphoma, blastoma, sarcoma, neuroendocrine tumor, mesothelioma, schwannoma, meningioma, adenocarcinoma, or melanoma. The cancer is not limited thereto and includes any cancer that overexpresses EGFR or PSMA on the surface of cancer cells.

Since the pharmaceutical composition of the present disclosure uses, as an active ingredient, the protein complex comprising the antibody or antigen-binding fragment thereof and the affibodies of the present disclosure as described above, the overlapping content between the two is omitted herein to avoid undue complexity of the description.

In one embodiment of the present disclosure, the cancer is selected from the group consisting of breast cancer, ovarian cancer, gastric cancer, lung cancer, non-small cell lung cancer, liver cancer, hepatocellular carcinoma, bronchial cancer, nasopharyngeal cancer, laryngeal cancer, pancreatic cancer, bladder cancer, colorectal cancer, colon cancer, rectal cancer, cervical cancer, brain cancer, prostate cancer, bone cancer, head and neck cancer, skin cancer, thyroid cancer, parathyroid cancer, squamous cell carcinoma, peritoneal cancer, kidney cancer, testicular cancer, esophageal cancer, and ureteral cancer; however, it is not limited thereto, and the cancer may be, for example, a carcinoma, lymphoma, blastoma, sarcoma, neuroendocrine tumor, mesothelioma, schwannoma, meningioma, adenocarcinoma, or melanoma. The cancer is not limited thereto and includes any cancer that overexpresses EGFR or PSMA on the surface of cancer cells.

The pharmaceutically acceptable carrier included in the pharmaceutical composition of the present disclosure is one commonly used in formulation, and includes, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, and mineral oil. The pharmaceutical composition of the present disclosure may additionally comprise lubricants, wetting agents, sweeteners, flavoring agents, emulsifiers, suspending agents, preservatives, and the like. Suitable pharmaceutically acceptable carriers and formulations are described in detail in Remington's Pharmaceutical Sciences (19th ed., 1995).

The pharmaceutical composition of the present disclosure may be administered orally or parenterally, for example, by intravenous injection, subcutaneous injection, intramuscular injection, intraperitoneal injection, intrasternally, topical administration, intranasal administration, intrapulmonary administration, or rectal administration.

An appropriate dosage of the pharmaceutical composition of the present disclosure varies depending on factors such as the formulation method, mode of administration, age, weight, sex, pathological condition, diet, administration time, administration route, excretion rate, and responsiveness of the patient, and a skilled physician can readily determine and prescribe a dosage effective for the desired treatment or prevention. In a preferred embodiment of the present disclosure, the daily dose of the pharmaceutical composition is 0.0001-100 mg/kg. As used herein, the term "therapeutically effective amount" refers to an amount sufficient to prevent or treat the above-described disease.

As used herein, the term "prevention" refers to prophylactic treatment or protection against a disease or disease condition. The term "treatment" refers to the reduction, suppression, alleviation, or elimination of a disease condition.

The pharmaceutical composition of the present disclosure may be prepared in a unit dosage form or filled into a multi-dose container by formulating, in a manner readily practiced by a person skilled in the art to which the present disclosure pertains, using pharmaceutically acceptable carriers and/or excipients. The formulation may be in the form of a solution, suspension, or emulsion in an oil or aqueous medium, or in the form of an extract, powder, suppository, powder preparation, granule, tablet, or capsule, and may additionally comprise a dispersant or stabilizer.

In one embodiment of the present disclosure, the dual antigen targeting protein complex is one in which an affibody dimer is bound to the C-terminus of the heavy chain of the antibody or antigen-binding fragment thereof.

In a particular embodiment of the present disclosure, the dual antigen targeting protein complex is one in which an affibody dimer is bound to the C-terminus of the heavy chain of the antibody or antigen-binding fragment thereof:
the heavy chain of the antibody or antigen-binding fragment thereof consisting of the amino acid sequence of SEQ ID NO: 9, SEQ ID NO: 33, or SEQ ID NO: 57; and
the affibody consisting of the amino acid sequence of SEQ ID NO: 1 or SEQ ID NO: 2.

In one embodiment of the present disclosure, the dual antigen targeting protein complex is one in which an affibody monomer is bound to the C-terminus of each of the heavy chain and the light chain of the antibody or antigen-binding fragment thereof.

In a particular embodiment of the present disclosure, the dual antigen targeting protein complex is one in which an affibody monomer is bound to the C-terminus of each of the heavy chain and the light chain of the antibody or antigen-binding fragment thereof:
the heavy chain of the antibody or antigen-binding fragment thereof consisting of the amino acid sequence of SEQ ID NO: 9, SEQ ID NO: 33, or SEQ ID NO: 57;
the light chain of the antibody or antigen-binding fragment thereof consisting of the amino acid sequence of SEQ ID NO: 10, SEQ ID NO: 34, or SEQ ID NO: 58; and
the affibody consisting of the amino acid sequence of SEQ ID NO: 1 or SEQ ID NO: 2.

In one embodiment of the present disclosure, the dual antigen targeting protein complex is one in which an affibody dimer is bound to the C-terminus of the light chain of the antibody or antigen-binding fragment thereof.

In a particular embodiment of the present disclosure, an affibody dimer is bound to the C-terminus of the light chain of the antibody or antigen-binding fragment thereof:
the light chain of the antibody or antigen-binding fragment thereof **consisting of** the amino acid sequence of SEQ ID NO: 10, SEQ ID NO: 34, or SEQ ID NO: 58; and
the affibody consisting of the amino acid sequence of SEQ ID NO: 1 or SEQ ID NO: 2.

In one embodiment of the present disclosure, the dual antigen targeting protein complex comprises an affibody monomer-bound heavy chain consisting of the amino acid sequence of SEQ ID NO: 11 and a light chain consisting of the amino acid sequence of SEQ ID NO: 12; an affibody monomer-bound heavy chain consisting of the amino acid sequence of SEQ ID NO: 19 and a light chain consisting of the amino acid sequence of SEQ ID NO: 20; an affibody monomer-bound heavy chain consisting of the amino acid sequence of SEQ ID NO: 35 and a light chain consisting of the amino acid sequence of SEQ ID NO: 36; an affibody monomer-bound heavy chain consisting of the amino acid sequence of SEQ ID NO: 43 and a light chain consisting of the amino acid sequence of SEQ ID NO: 44; an affibody monomer-bound heavy chain consisting of the amino acid sequence of SEQ ID NO: 59 and a light chain consisting of the amino acid sequence of SEQ ID NO: 60; or an affibody monomer-bound heavy chain consisting of the amino acid sequence of SEQ ID NO: 67 and a light chain consisting of the amino acid sequence of SEQ ID NO: 68.

In one embodiment of the present disclosure, the dual antigen targeting protein complex comprises an affibody dimer-bound heavy chain consisting of the amino acid sequence of SEQ ID NO: 13 and a light chain consisting of the amino acid sequence of SEQ ID NO: 14; an affibody dimer-bound heavy chain consisting of the amino acid sequence of SEQ ID NO: 21 and a light chain consisting of the amino acid sequence of SEQ ID NO: 22; an affibody dimer-bound heavy chain consisting of the amino acid sequence of SEQ ID NO: 37 and a light chain consisting of the amino acid sequence of SEQ ID NO: 38; an affibody dimer-bound heavy chain consisting of the amino acid sequence of SEQ ID NO: 45 and a light chain consisting of the amino acid sequence of SEQ ID NO: 46; an affibody dimer-bound heavy chain consisting of the amino acid sequence of SEQ ID NO: 61 and a light chain consisting of the amino acid sequence of SEQ ID NO: 62; or an affibody dimer-bound heavy chain consisting of the amino acid sequence of SEQ ID NO: 69 and a light chain consisting of the amino acid sequence of SEQ ID NO: 70.

In one embodiment of the present disclosure, the dual antigen targeting protein complex comprises an affibody monomer-bound heavy chain consisting of the amino acid sequence of SEQ ID NO: 15 and an affibody monomer-bound light chain consisting of the amino acid sequence of SEQ ID NO: 16; an affibody monomer-bound heavy chain consisting of the amino acid sequence of SEQ ID NO: 23 and an affibody monomer-bound light chain consisting of the amino acid sequence of SEQ ID NO: 24; an affibody monomer-bound heavy chain consisting of the amino acid sequence of SEQ ID NO: 39 and an affibody monomer-bound light chain consisting of the amino acid sequence of SEQ ID NO: 40; an affibody monomer-bound heavy chain consisting of the amino acid sequence of SEQ ID NO: 47 and an affibody monomer-bound light chain consisting of the amino acid sequence of SEQ ID NO: 48; an affibody monomer-bound heavy chain consisting of the amino acid sequence of SEQ ID NO: 63 and an affibody monomer-bound light chain consisting of the amino acid sequence of SEQ ID NO: 64; or an affibody monomer-bound heavy chain consisting of the amino acid sequence of SEQ ID NO: 71 and an affibody monomer-bound light chain consisting of the amino acid sequence of SEQ ID NO: 72.

In one embodiment of the present disclosure, the dual antigen targeting protein complex comprises a heavy chain consisting of the amino acid sequence of SEQ ID NO: 17 and an affibody dimer-bound light chain consisting of the amino acid sequence of SEQ ID NO: 18; a heavy chain consisting of the amino acid sequence of SEQ ID NO: 25 and an affibody dimer-bound light chain consisting of the amino acid sequence of SEQ ID NO: 26; a heavy chain consisting of the amino acid sequence of SEQ ID NO: 41 and an affibody dimer-bound light chain consisting of the amino acid sequence of SEQ ID NO: 42; a heavy chain consisting of the amino acid sequence of SEQ ID NO: 49 and an affibody dimer-bound light chain consisting of the amino acid sequence of SEQ ID NO: 50; a heavy chain consisting of the amino acid sequence of SEQ ID NO: 65 and an affibody dimer-bound light chain consisting of the amino acid sequence of SEQ ID NO: 66; or a heavy chain consisting of the amino acid sequence of SEQ ID NO: 73 and an affibody dimer-bound light chain consisting of the amino acid sequence of SEQ ID NO: 74.

In one aspect of the present disclosure, there is provided a method for preventing or treating cancer, comprising administering to a subject a dual antigen targeting protein complex comprising an EGFR (Epidermal Growth Factor Receptor) or PSMA (Prostate-Specific Membrane Antigen) targeting domain comprising an antibody or an antigen-binding fragment thereof, and a CD137 targeting domain comprising an affibody bound to the antibody or the antigen-binding fragment thereof, or a pharmaceutical composition comprising the same.

Since the cancer prevention or treatment method of the present disclosure involves administering the dual antigen targeting protein complex or a pharmaceutical composition comprising the same to a subject, common matters relating to the dual antigen targeting protein complex or the pharmaceutical composition comprising the same are omitted herein in order to avoid undue complexity of the present specification.

### Advantageous Effects of Invention

The features and advantages of the present disclosure may be summarized as follows:
(a) The present disclosure provides a dual antigen targeting protein complex comprising: a first antigen targeting domain comprising an antibody or an antigen-binding fragment thereof; and a second antigen targeting domain comprising affibodies bound to the antibody or the antigen-binding fragment thereof.
(b) The present disclosure provides a nucleic acid molecule comprising a nucleotide sequence encoding the dual antigen targeting protein complex.
(c) The present disclosure provides a recombinant vector comprising the nucleic acid molecule.
(d) The present disclosure provides a host cell comprising the recombinant vector.
(e) The present disclosure provides a pharmaceutical composition for preventing or treating cancer, comprising a dual antigen targeting protein complex comprising: an EGFR (Epidermal Growth Factor Receptor) or PSMA (Prostate-Specific Membrane Antigen) targeting domain comprising an antibody or an antigen-binding fragment thereof; and a CD137 targeting domain comprising affibodies bound to the antibody or the antigen-binding fragment thereof.
(f) The present disclosure provides a method for preventing or treating cancer, comprising administering to a subject a dual antigen targeting protein complex comprising: an EGFR (Epidermal Growth Factor Receptor) or PSMA (Prostate-Specific Membrane Antigen) targeting domain comprising an antibody or an antigen-binding fragment thereof; and a CD137 targeting domain comprising affibodies bound to the antibody or the antigen-binding fragment thereof, or a pharmaceutical composition comprising the same.
(g) By using the dual antigen targeting protein complex of the present disclosure or a composition comprising the same, cancer can be effectively prevented or treated.

### Brief Description of Drawings

FIG. 1 is a schematic representation of an optimized form of the dual antigen targeting protein complex.
FIG. 2 shows the results of producing various forms of the dual antigen targeting protein complex based on cetuximab in animal cells and confirming successful production through SDS-PAGE.
FIG. 3a shows a comparison of the binding affinities of CET_ZAAD01D and CET_ZAAD01M4 to CD137 and EGFR proteins.
FIG. 3b shows a comparison of the binding affinities of CET_ZAAD05D and CET_ZAAD05M4 to CD137 and EGFR proteins.
FIG. 3c shows a comparison of the binding affinities of CET_ZAAD01D and CET_ZAAD01D_LC to CD137 and EGFR proteins.
FIG. 3d shows a comparison of the binding affinities of CET_ZAAD05D and CET_ZAAD05D_LC to CD137 and EGFR proteins.
FIG. 4 shows a comparison of the binding affinities of various forms of the dual antigen targeting protein complex based on cetuximab to activated CEMT cells expressing CD137.
FIG. 5a shows a comparison of the anticancer effects of various forms of the dual antigen targeting protein complex, CET_ZAAD01D, CET_ZAAD01M4, CET_ZAAD05D, and CET_ZAAD05M4, based on cetuximab through cytotoxicity testing.
FIG. 5b shows a comparison of the anticancer effects of various forms of the dual antigen targeting protein complex, CET_ZAAD01D, CET_ZAAD01D_LC, ZAAD05D, and CET_ZAAD05D_LC, based on cetuximab through cytotoxicity testing.
FIGS. 6a and 6b show a comparison of the ability of various forms of the dual antigen targeting protein complex based on cetuximab to activate EGFR protein-dependent CD8 T cells.
FIG. 7 shows a comparison of the ability of various forms of the dual antigen targeting protein complex based on cetuximab to activate HT29 cell-dependent CD8 T cells.
FIGS. 8a, 8b, 8c, and 8d show the results of comparing the purity of various forms of the dual antigen targeting protein complex based on cetuximab through SEC-HPLC.
FIG. 9 shows the results of confirming the anticancer effects of various forms of the dual antigen targeting protein complex based on 15E3 through cytotoxicity testing.
FIG. 10 shows the results of producing a dual antigen targeting protein complex based on a PSMA-targeting antibody and confirming it through SDS-PAGE.
FIG. 11 shows the results of analyzing the ability of a dual antigen targeting protein complex based on a PSMA-targeting antibody to activate LNCap cell-dependent CD8 T cells.

### Best Mode for Carrying out the Invention

Hereinafter, the present disclosure will be described in further detail through the following Examples. These Examples are provided solely for the purpose of illustrating the present disclosure in greater detail, and it will be apparent to those of ordinary skill in the art that the scope of the present disclosure is not limited by these Examples, but may be embodied in other forms in accordance with the spirit of the present disclosure.

### Examples

### Example 1: Production of Various Forms of Bispecific Antigen-Targeting Protein Complexes

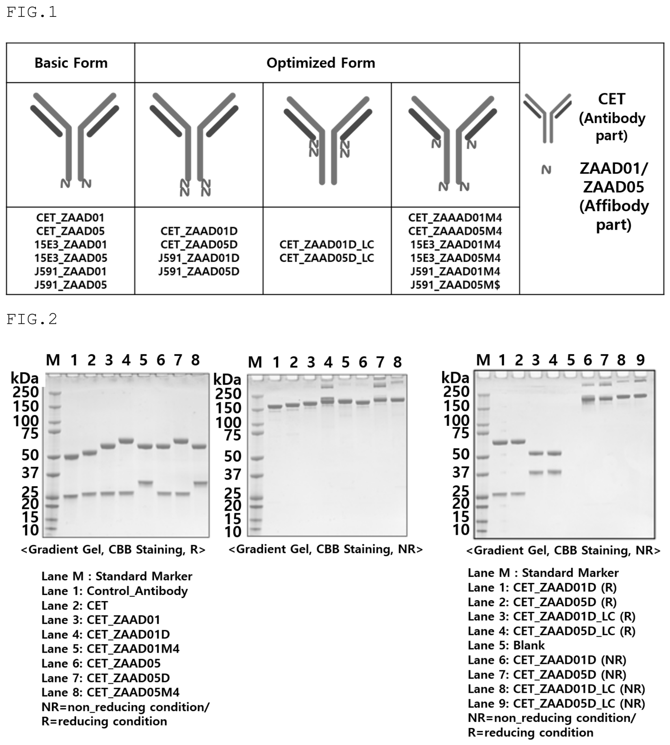

To produce bispecific antigen-targeting protein complexes, antibodies and affibodies were conjugated to form the protein complexes. To determine the optimal form of the produced protein complexes, the configurations shown in FIG. 1 were obtained through gene cloning. The resulting protein complexes were expressed in animal cells, and their size and purity were confirmed by SDS-PAGE analysis.

HEK293F cells were prepared at a density of 1 × 10⁶ cells/mL in 100 mL culture medium and cultured under shaking conditions at 37 °C, 8% CO₂, and 125 rpm. In a sterile 15 mL tube, 5 mL of culture medium was mixed with expression vector DNA encoding the heavy chain and light chain of the antibody to prepare the DNA mixture. Polyethylenimine (PEI; Polysciences, cat. no. 23966) was added to the tube containing culture medium and DNA, mixed, and then added to the HEK293F cells, followed by incubation for seven days. Recombinant proteins in the culture supernatant were purified using Protein A resin (GE Healthcare, cat. no. 17-5438-03) and buffer-exchanged by diafiltration (Sartorius, cat. no. VS2002). The purified recombinant proteins were quantified by measuring absorbance at 280 nm and analyzed by SDS-PAGE using a 4-20% gradient gel.

Using the form in which an affibody is conjugated to the C-terminus of the antibody heavy chain as the base configuration, various optimized bispecific antigen-targeting protein complexes were produced. The form in which an affibody dimer is additionally conjugated to the C-terminus of the antibody heavy chain was designated as the "D form," the form in which affibodies are additionally conjugated to the C-termini of both the antibody heavy and light chains was designated as the "M4 form," and the form in which an affibody dimer is additionally conjugated to the C-terminus of the antibody light chain was designated as the "D_LC form." The structures of the produced bispecific antigen-targeting protein complexes are shown in FIG. 1.

### Example 2: Production of Various Forms of Bispecific Antigen-Targeting Protein Complexes Based on EGFR-Targeting Antibody (Cetuximab)

The bispecific antigen-targeting protein complexes designed as described above were produced using the cetuximab antibody and affibodies (ZAAD01/ZAAD05) that bind to CD137. The bispecific antigen-targeting protein complexes in the D form, M4 form, and D_LC form, prepared using cetuximab and the affibodies, were designated as CET_ZAAD01D/CET_ZAAD05D, CET_ZAAD01M4/CET_ZAAD05M4, and CET_ZAAD01D_LC/CET_ZAAD05D_LC, respectively.

SDS-PAGE analysis was performed to verify whether each bispecific antibody was successfully produced at the intended molecular size.

The results are shown in FIG. 2

As shown in FIG. 2, compared with CET, the heavy chain bands with increased molecular weight were observed in CET_ZAAD01 and CET_ZAAD05. Compared with CET_ZAAD01 and CET_ZAAD05, the heavy chain bands with further increased molecular weight were observed in CET _ZAAD01D and CET_ZAAD05D. Compared with CET, both the heavy and light chain bands with increased molecular weight were observed in CET_ZAAD01M4 and CET_ZAAD05M4. Compared with CET_ZAAD01D and CET_ZAAD05D, CET_ZAAD01D_LC and CET_ZAAD05D_LC showed heavy chain bands with decreased molecular weight and light chain bands with increased molecular weight. These results confirmed that each form of bispecific antibody was successfully produced under reducing conditions (FIG. 2). For some of the optimized bispecific antibody candidates (CET_ZAAD01D, CET_ZAAD05D, and CET_ZAAD05D_LC), bands with larger sizes than the expected molecular weight were observed under non-reducing conditions (FIG. 2).

### Example 3: Comparison of Target-Binding Ability of Bispecific Antigen-Targeting Protein Complexes Based on EGFR-Targeting Antibody (Cetuximab)

The binding affinity of the purified bispecific antigen-targeting protein complexes to the target proteins or to cells expressing the target proteins was analyzed. Using eight types of the bispecific antigen-targeting protein complexes produced, the binding to CD137 and EGFR proteins was evaluated by ELISA. In the ELISA, plates coated with hCD137-ECD-Fc and hEGFR-ECD-Fc proteins at a concentration of 1 µg/mL were treated with each of the purified bispecific antibodies, serially diluted from 60 nM in 1:5 steps to generate seven concentration points. After treatment with the secondary antibody (anti-hIgG-Fab-HRP; Jackson, JAC-109-035-097), a colorimetric reaction was developed using TMB (biofx, TMBC-1000-01), and the OD450 value was measured using an ELISA reader (Victor X3, PerkinElmer). EC50 values were calculated using GraphPad Prism.

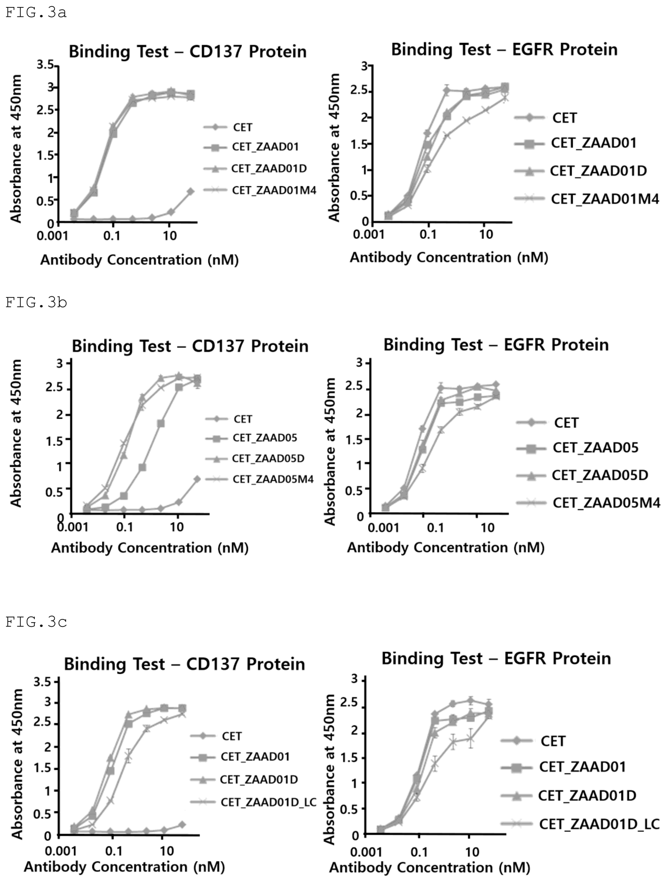

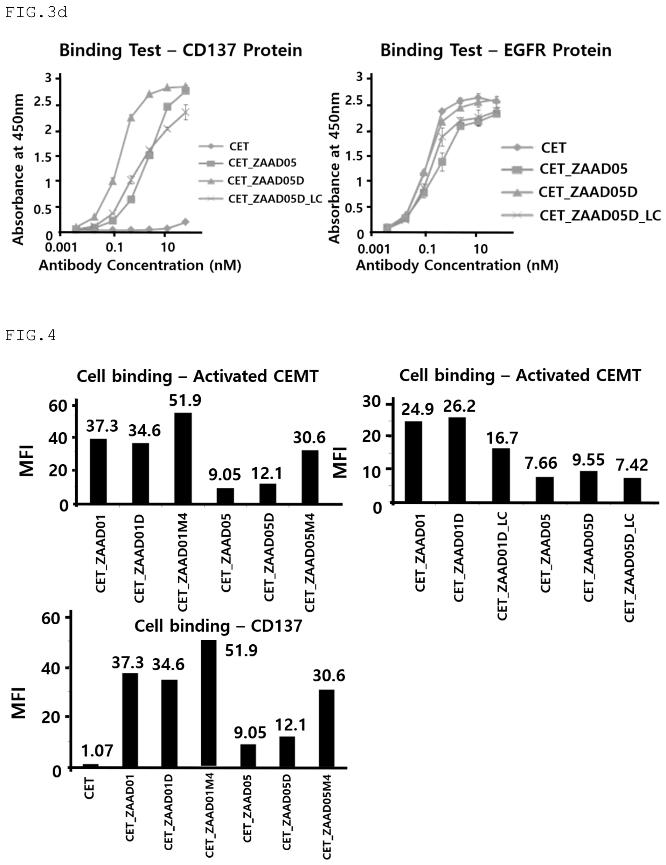

The results are shown in FIGS. 3a, 3b, 3c, 3d, Table 1, and Table 2.

As shown in FIG. 3a, CET_ZAAD01, CET_ZAAD01D, and CET_ZAAD01M4 exhibited no significant difference in binding to the CD137 protein. Consistently, the EC50 values listed in Table 1 also showed no substantial difference. As shown in FIG. 3b, CET_ZAAD05D and CET_ZAAD05M4 demonstrated increased binding to the CD137 protein compared with CET_ZAAD05, and the EC50 values in Table 1 confirmed more than a ten-fold improvement.

**Table 1.**

| Binding affinity analysis results of CET_ZAAD01D, CET_ZAAD01M4, CET_ZAAD05D, and CET_ZAAD05M4 for CD137 protein and EGFR protein. | | |
|---|---|---|
| | **EC50** | |
| | **CD137** | **EGFR** |
| **CET_ZAAD01** | 0.055 | 0.073 |
| **CET**_**ZAAD01D** | 0.047 | 0.100 |
| **CET_ZAAD01M4** | 0.046 | 0.121 |
| **CET**_**ZAAD05** | 1.194 | 0.087 |
| **CET_ZAAD05D** | 0.128 | 0.086 |
| **CET_ZAAD05M4** | 0.092 | 0.155 |

As shown in FIGS. 3c and 3d and Table 2, CET_ZAAD01D_LC and CET_ZAAD05D_LC showed either reduced or similar binding compared with CET_ZAAD01 and CET_ZAAD05, respectively.

**Table 2.**

| Binding affinity analysis results of CET_ZAAD01D, CET_ZAAD01_LC, CET_ZAAD05D, and CET_ZAAD05_LC for CD137 protein and EGFR protein. | | |
|---|---|---|
| | **EC50** | |
| | **CD137** | **EGFR** |
| **CET_ZAAD01** | 0.099 | 0.110 |
| **CET**_**ZAAD01D** | 0.074 | 0.147 |
| **CET_ZAAD01D_LC** | 0.258 | 0.236 |
| **CET_ZAAD05** | 2.231 | 0.263 |
| **CET_ZAAD05D** | 0.169 | 0.114 |
| **CET**_**ZAAD05D_LC** | 0.867 | 0.150 |

To evaluate the binding ability of various forms of the bispecific antigen-targeting protein complexes to CD137-expressing cells, CEMT cells (ATCC, CCL-119) were prepared at 1 × 10⁷ cells/10 mL and activated by treatment with 50 ng/mL PMA (Sigma, P1585) and 1 µg/mL ionomycin (Sigma, I9657) for 16 hours. Activated CEMT cells were prepared at a concentration of 5 × 10⁵ cells/tube, centrifuged at 1200 rpm for 3 minutes to pellet the cells, and washed with PBS containing 5% FBS. The bispecific antigen-targeting protein complex was then added at a concentration of 0.48 nM, followed by incubation on ice for 1 hour. Cells were washed three times with 200 µL of PBS containing 5% FBS by centrifugation at 1200 rpm for 3 minutes. Anti-human Fc-FITC (LT, A11013) was then added at 1 µg/mL, and the cells were incubated on ice in the dark for 45 minutes. After three washes with 200 µL of PBS containing 5% FBS using the same centrifugation conditions, fluorescence intensity was measured using a Beckman Coulter FACS instrument.

The results are shown in FIG. 4.

As shown in FIG. 4, when assessing binding to activated CEMT cells, CET_ZAAD01D showed a similar MFI value to CET_ZAAD01, CET_ZAAD01M4 showed an increased MFI value, and CET_ZAAD01D_LC showed a decreased MFI value. Compared with CET_ZAAD05, CET_ZAAD05D showed a similar MFI value, CET_ZAAD05M4 showed an increased MFI value, and CET_ZAAD05D_LC showed a similar MFI value.

### Example 4: Comparison of Cytotoxicity of Bispecific Antigen-Targeting Protein Complexes Based on an EGFR-Targeting Antibody (Cetuximab)

To evaluate the potential anti-cancer effects, the cytotoxicity of the produced bispecific antigen-targeting protein complexes was compared under co-culture conditions of PBMCs and EGFR-expressing cells. One day prior, 96-well round-bottom plates were coated with 50 µL per well of an anti-CD3 antibody (Invitrogen, 16-0037-85) at 5 µg/mL. The plates were washed three times with 100 µL of culture medium (RPMI1640, 10% FBS), followed by blocking with 100 µL of the same medium at 37°C for 1 hour. After removing the blocking medium, DLD-1 cells expressing luciferase were added at 50 µL/well at a concentration of 1.0 × 10⁵ cells/mL. Eight types of bispecific antigen-targeting protein complexes were added at concentrations starting from 1 nM, serially diluted 1:10 over six points (50 µL/well). Meanwhile, blood obtained from a healthy donor was diluted 1:2 with PBS and layered onto a Leucosep tube (Greiner bio-one, 227290) containing Ficoll (GE Healthcare, 17-1440-12), followed by centrifugation at 1000 × g for 30 minutes. The PBMC layer above the Ficoll was collected and washed twice with PBS containing 2% FBS at 300 × g for 10 minutes. PBMCs were counted and added to the wells at 50 µL/well at 1.0 × 10⁵ cells/mL, followed by incubation for 3 days. After incubation, the plates were centrifuged at 2000 rpm for 2 minutes. Fifty µL of supernatant from each well was transferred to a 96-well V-bottom plate for subsequent experiments. To the remaining wells, 50 µL/well of 3× lysis buffer (75 mM Tris, pH 8.0, 30% glycerol, 3% Triton X-100) was added, mixed, and incubated at room temperature for 15 minutes. Lysates were transferred to a 96-well white plate, followed by addition of 50 µL/well of luciferase assay reagent (Promega, G7940), mixing, and incubation in the dark at room temperature for 15 minutes. Luminescence was then measured using the VICTOR^{™} X3 instrument.

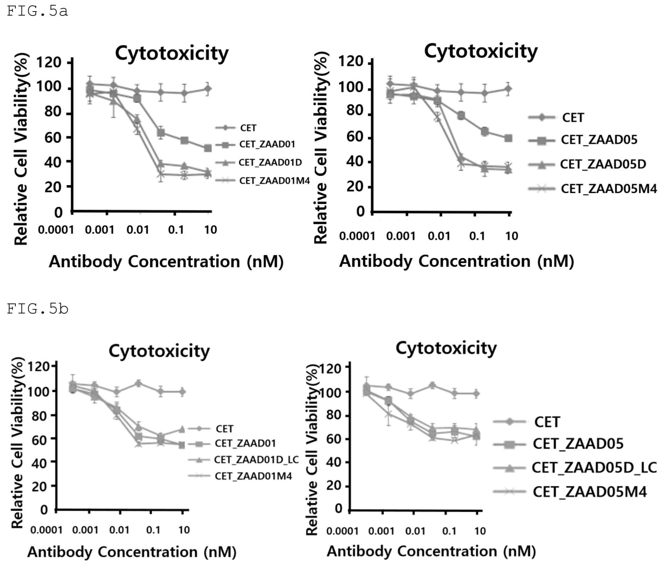

The results are shown in FIGs. 5a and 5b.

As shown in FIGs. 5a and 5b, cytotoxicity testing revealed that the D form, M4 form, and D_LC form of the protein complexes exhibited increased cytotoxicity compared to the basic form of the bispecific antigen-targeting protein complex. CET_ZAAD01D and CET_ZAAD01M4 demonstrated enhanced cytotoxicity compared to CET_ZAAD01, with maximum cell death increasing from approximately 50% to about 70%. CET_ZAAD05D and CET_ZAAD05M4 also showed enhanced maximum cell death rates, increasing from about 40% to approximately 65% compared to CET_ZAAD05. CET_ZAAD01D_LC showed cytotoxicity comparable to CET_ZAAD01D, and CET_ZAAD05D_LC showed cytotoxicity comparable to CET_ZAAD05D.

### Example 5: Comparison of CD8 T Cell Activation Ability of Various Forms of Bispecific Antigen-Targeting Protein Complexes Based on an EGFR-Targeting Antibody (Cetuximab)

The ability of the various produced bispecific antigen-targeting protein complexes to activate CD8 T cells in an EGFR-dependent manner was evaluated. One day prior, 96-well round-bottom plates were coated with 50 µL/well of anti-CD3 antibody (Invitrogen, 16-0037-85) at 3 µg/mL. The next day, the plates were washed three times with 100 µL of sterile PBS. Human EGFR-ECD-Fc (hEGFR-ECD-Fc) was then coated at 5 µg/mL, 50 µL/well, and incubated at 37°C for 3 hours. The plates were washed three times with R10 medium (RPMI1640, 10% FBS, 10 mM HEPES) and blocked with 100 µL/well of the same medium at 37°C for 1 hour. After removing the blocking medium, six bispecific antibodies were serially diluted from 5 nM at a 1:5 ratio over four points and added at 50 µL/well for 30 minutes. Meanwhile, blood from a healthy donor was diluted 1:2 with PBS and layered onto a Leucosep tube (Greiner Bio-One, 227290) containing Ficoll (GE Healthcare, 17-1440-12), followed by centrifugation at 1000 × g for 30 minutes. The PBMC layer above the Ficoll was collected, washed twice with PBS containing 2% FBS at 300 × g for 10 minutes, and counted. CD8+ T cells were isolated from PBMCs using a CD8 isolation kit (Miltenyi Biotec, 130-096-495) according to the manufacturer's protocol. The CD8+ T cells were resuspended in R10 medium at 1.4 × 10⁶/mL and added to the bispecific antibody-treated plates at 50 µL/well. After 3 days, the plates were centrifuged at 2000 rpm for 10 minutes, and the supernatants were collected. IFN-γ levels were measured using an IFN-γ ELISA kit (BD, 555142). CD8 T cell activation was compared by measuring both CD8 T cell proliferation (WST-8 assay) and IFN-γ secretion.

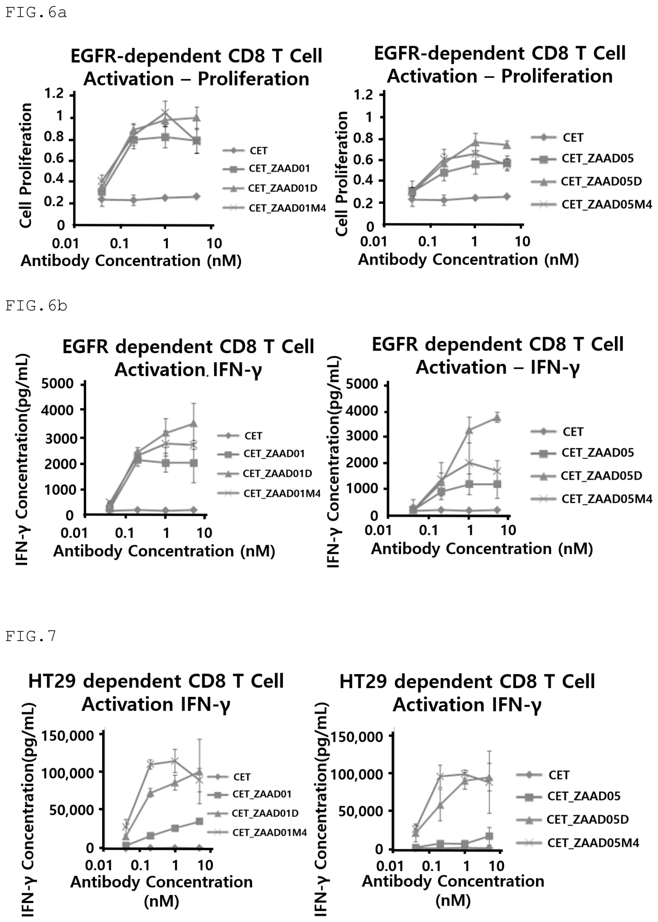

The results are shown in FIGs. 6a and 6b.

As shown in FIGs. 6a and 6b, the CD8 T cell activation ability was superior in the D form and M4 form compared to the basic form of the bispecific antigen-targeting protein complex. In FIG. 6a, CD8 T cell proliferation was increased in CET_ZAAD01D and CET_ZAAD01M4 compared to CET_ZAAD01, and in CET_ZAAD05D and CET_ZAAD05M4 compared to CET_ZAAD05. In FIG. 6b, IFN-γ secretion showed a similar trend to that observed for cell proliferation.

In addition, a CD8 T cell activation assay was performed using EGFR-expressing HT29 cells for comparison.

One day prior, 96-well round-bottom plates were coated with 50 µL/well of anti-CD3 antibody at 3 µg/mL. The next day, the plates were washed three times with 100 µL of sterile PBS, then washed three times with R10 medium and blocked with 100 µL/well of R10 medium at 37°C for 1 hour. After removing the blocking medium, HT29 cells were counted, resuspended in R10 medium at 7 × 10⁵/mL, and added at 50 µL/well. Six bispecific antigen-targeting protein complexes were serially diluted from 5 nM at a 1:5 ratio over four points and added at 50 µL/well for 30 minutes. In parallel, PBMCs were prepared from healthy donor blood as described above, and CD8+ T cells were isolated using a CD8 isolation kit. The CD8+ T cells were resuspended in R10 medium at 1.4 × 10⁶/mL and added to the bispecific antibody-treated plates at 50 µL/well. After 3 days, the plates were centrifuged at 2000 rpm for 10 minutes, and the supernatants were collected for IFN-γ measurement using the BD ELISA kit.

The results are shown in FIG. 7.

As shown in FIG. 7, the levels of secreted IFN-γ were markedly increased in CET_ZAAD01D and CET_ZAAD01M4 compared to CET_ZAAD01, and in CET_ZAAD05D and CET_ZAAD05M4 compared to CET_ZAAD05.

### Example 6: SEC-HPLC Analysis of Various Forms of Bispecific Antigen-Targeting Protein Complexes Based on an EGFR-Targeting Antibody (Cetuximab)

The purity of the produced bispecific antigen-targeting protein complexes was compared using SEC-HPLC analysis. For purity testing, each bispecific antigen-targeting protein complex was prepared at a concentration of 1 mg/mL and filtered through a 0.2 µm PES filter. The filtered samples were aliquoted at 20 µL each into HPLC screw vials. The HPLC system was connected to a mobile phase buffer (0.1 M sodium phosphate + 0.2 M arginine, pH 6.8) and an SEC-HPLC column (TOSOH, SWXL3000 - 7.8 mm × 30 cm). The method parameters were set to a flow rate of 0.8 mL/min, detection at 280 nm, injection volume of 10 µL, and temperature of 25°C. The system's analysis program was configured with the sample sequence and sample names according to the number of samples to be analyzed, and the instrument was run to analyze each bispecific antigen-targeting protein complex. The HPLC analysis software was used to determine the retention time of each sample and the area of the peaks.

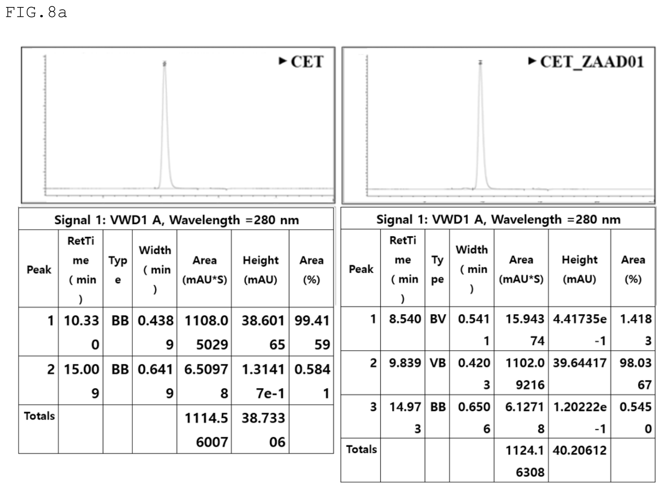

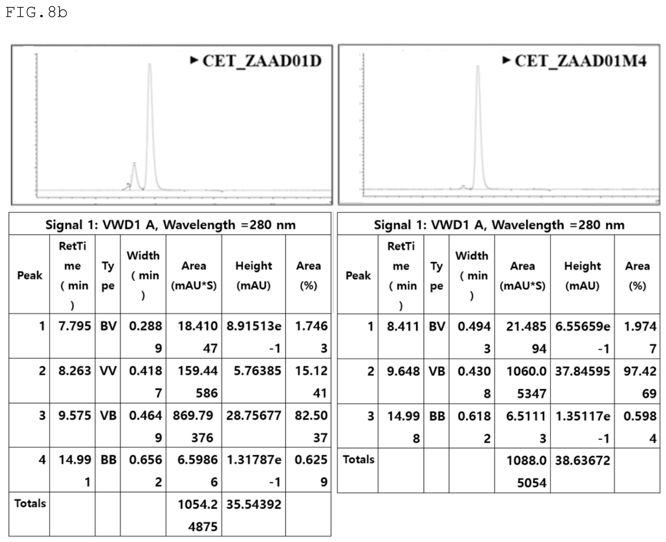

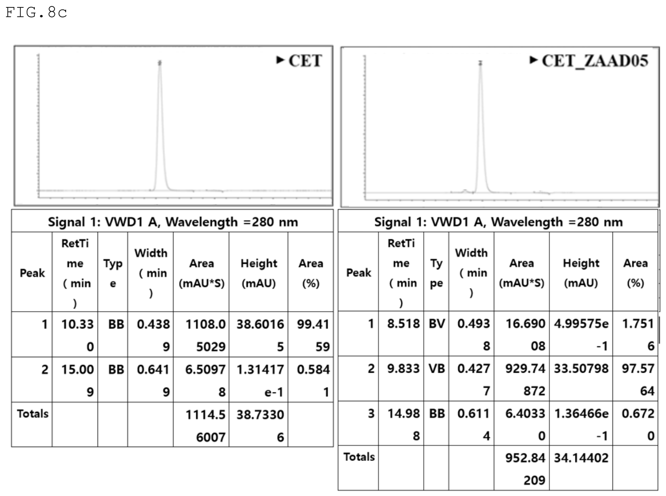

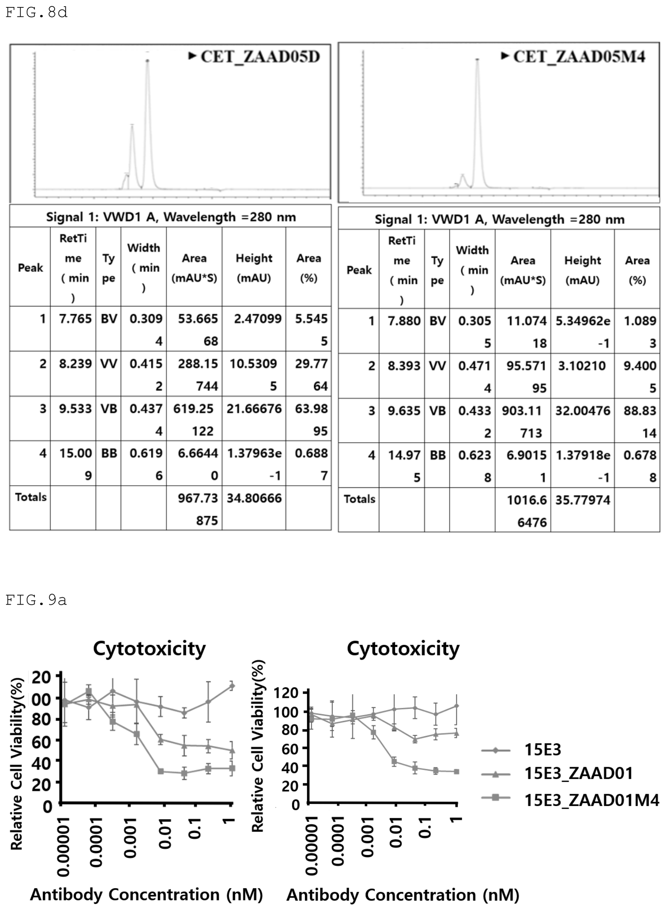

The results are shown in FIGs. 8a, 8b, 8c, 8d and Table 3.

As shown in FIGs. 8a, 8b, 8c, 8d and Table 3, SEC-HPLC purity analysis was performed for six optimized bispecific antibody forms. The retention times of the main peaks were confirmed in comparison with CET, and the corresponding purities were calculated. CET_ZAAD01 showed a purity of 98%, CET_ZAAD01D approximately 83%, and CET_ZAAD01M4 approximately 97%. CET_ZAAD05 showed a purity of approximately 97%, CET_ZAAD05D approximately 64%, and CET_ZAAD05M4 approximately 89%.

**Table 3.**

| SEC-HPLC main peak purity percentages | | |
|---|---|---|
| | **Main Peak** | |
| | **Retention time (min)** | **Area percentage (%)** |
| **CET** | 10.33 | 99.41 |
| **CET_ZAAD01** | 9.84 | 98.03 |
| **CET**_**ZAAD01D** | 9.58 | 82.50 |
| **CET_ZAAD01M4** | 9.65 | 97.42 |
| **CET_ZAAD05** | 9.83 | 97.58 |
| **CET**_**ZAAD05D** | 9.53 | 63.99 |
| **CET**_**ZAAD05M4** | 9. 64 | 88.83 |

The overall evaluation results of various forms of bispecific antigen-targeting protein complexes based on cetuximab are shown in Table 4.

**Table 4.**

| Comprehensive evaluation results of cetuximab-based bispecific antigen-targeting protein complexes | | | | | | | | | | | |
|---|---|---|---|---|---|---|---|---|---|---|---|
| | **Protein binding** | | | | **Cell binding** | | **Cytotoxici ty** | | **CD8 T cell activation** | | |
| | **EC50 (nM)** | | | | **MFI** | | **Maxinal activity (% )** | | **Maxinal activity (O.D, pg/mL, pg/mL)** | | |
| | **1st** | | **2nd** | | **1st** | **2nd** | **1st** | **2nd** | **Proliferat ion** | **IFN-Gamma** | **IFN-Gamma** |
| | **CD13 7** | **EGF R** | **CD13 7** | **EGF R** | **Activated CEMT** | | **DLD-1** | | **EFGR protein dependent** | | **HT29 cell depende nt** |
| **CET_ZAAD01** | 0.06 | 0.0 7 | 0.10 | 0.1 1 | 37.3 0 | 24.9 0 | 48.4 9 | N/A | 0.81 | 2031. 47 | 36649.5 0 |
| **CET_ZAAD01D** | 0.05 | 0.1 0 | 0.07 | 0.1 5 | 34.6 0 | 26.2 0 | 67.6 6 | 46.6 0 | 0.98 | 3580. 71 | 101752. 55 |
| **CET_ZAAD01M 4** | 0.05 | 0.1 2 | N/A | | 51.9 0 | N/A | 69.3 6 | 46.1 0 | 1.03 | 2784. 59 | 115571. 80 |
| **CET_ZAAD01D _Lc** | N/A | | 0.26 | 0.2 4 | N/A | 16.7 0 | N/A | 38.8 0 | N/A | N/A | N/A |
| **CET_ZAAD05** | 1.19 | 0.0 9 | 2.23 | 0.2 6 | 9.05 | 7.66 | 40.3 3 | N/A | 0.57 | 1195. 90 | 17418.3 6 |
| **CET_ZAAD05D** | 0.13 | 0.0 9 | 0.17 | 0.1 1 | 12.1 0 | 9.55 | 65.7 0 | 38.4 0 | 0.76 | 3809. 25 | 94241.9 0 |
| **CET_ZAAD05M 4** | 0.09 | 0.1 6 | N/A | | 30.6 0 | N/A | 63.4 7 | 41.7 0 | 0.65 | 2032. 21 | 98540.2 9 |
| **CET_ZAAD05D _Lc** | N/A | | 0.87 | 0.1 5 | N/A | 7.42 | N/A | 32.7 0 | N/A | N/A | N/A |

### Example 7: Comparison of Cytotoxicity of Various Forms of Bispecific Antigen-Targeting Protein Complexes Based on EGFR-Targeting Antibody (15E3)

Bispecific antigen-targeting protein complexes based on the EGFR-specific antibody 15E3 were prepared using the same method as described in Example 1. The potential anticancer effect was evaluated by comparing the cytotoxicity under co-culture conditions of PBMCs and EGFR-expressing cells. One day prior to the assay, anti-CD3 antibody (Invitrogen, 16-0037-85) was coated onto a 96-well round plate at 5 µg/mL, 50 µL/well. The plate was washed three times with 100 µL of culture medium (RPMI1640, 10% FBS) and then blocked with 100 µL/well of the same culture medium at 37 °C for 1 hour. After removing the blocking medium, DLD-1 cells expressing luciferase were prepared at 1.0 × 10⁵ cells/mL and seeded at 50 µL/well. Eight types of bispecific antibodies were serially diluted from 1 nM in 1:10 steps to obtain six concentration points, and 50 µL of each dilution was added. Meanwhile, whole blood from healthy donors was diluted 1:2 in PBS and layered onto a Leucosep tube (Greiner bio-one, 227290) containing Picoll (GE Healthcare, 17-1440-12). The samples were centrifuged at 1000 g for 30 minutes, and the PBMC layer was collected. PBMCs were washed twice with 2% PBS by centrifugation at 300 g for 10 minutes, counted, resuspended in culture medium at 1.0 × 10⁵ cells/mL, and added to the wells (50 µL/well). Co-cultures were incubated for 3 days. After incubation, the plates were centrifuged at 2000 RPM for 2 minutes to pellet the cells. Supernatants (50 µL/well) were transferred to 96-well V-bottom plates for later use. To the remaining plates, 50 µL/well of 3× lysis buffer (75 mM Tris, pH 8.0; 30% glycerol; 3% Triton X-100) was added and mixed, followed by incubation at room temperature for 15 minutes. Lysates were transferred to 96-well white plates (50 µL/well), and luciferase assay reagent (Promega, G7940) was added at 50 µL/well. The plates were mixed, incubated for 15 minutes at room temperature in the dark, and luminescence was measured using a VITOR^{™} X3 instrument. Two different donors were tested.

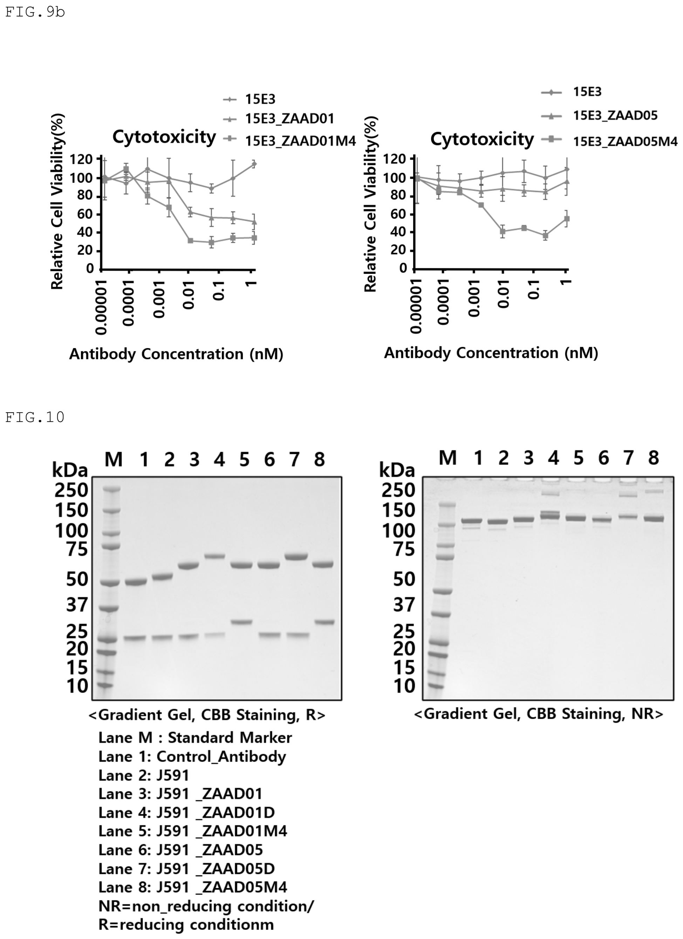

The results are shown in Figures 9a and 9b.

As shown in Figures 9a and 9b, analysis of cytotoxicity revealed that the M4 form of the protein complex exhibited superior cytotoxicity compared to the basic form of the bispecific antigen-targeting protein complex.

As shown in Figure 9a, cytotoxicity of 15E3_ZAAD01M4 increased compared to 15E3_ZAAD01. In donor 1, maximum cell viability decreased from approximately 50% to about 30%, indicating enhanced cytotoxicity. In donor 2, maximum cell viability decreased from approximately 70% to about 35%, confirming improved cytotoxicity.

As shown in Figure 9b, cytotoxicity of 15E3_ZAAD05M4 increased compared to 15E3_ZAAD05. In donor 1, maximum cell viability decreased from approximately 60% to about 35%, and in donor 2, from approximately 85% to about 35%, confirming enhanced cytotoxicity.

### Example 8: Production of Various Forms of Bispecific Antigen-Targeting Protein Complexes Based on a PSMA-Targeting Antibody

Various forms of bispecific antigen-targeting protein complexes based on a PSMA-targeting antibody were prepared using the J591 antibody and affibodies (ZAAD01/ZAAD05) in the same manner as described in Example 1.

Using the PSMA-targeting antibody J591, two basic forms, J591_ZAAD01 and J591_ZAAD05, each comprising one of the two affibody types, were first generated. From these basic forms, a D form, in which an affibody dimer was added to the C-terminus of the heavy chain, and an M4 form, in which affibodies were added to the C-termini of both the heavy and light chains, were produced. These were designated J591_ZAAD01D/J591_ZAAD05D and J591_ZAAD01M4/J591_ZAAD05M4, respectively.

As shown in Figure 10, SDS-PAGE analysis confirmed that each form of the bispecific antigen-targeting protein complex was successfully produced (reducing condition in Figure 10). Among the optimized bispecific antibody candidates, J591_ZAAD01D and J591_ZAAD05D displayed a noticeably larger band than the expected size under non-reducing conditions, which was consistent with the results observed for cetuximab-based antibodies.

### Example 9: Comparison of CD8 T Cell Activation Ability of Various Forms of Bispecific Antigen-Targeting Protein Complexes Based on a PSMA-Targeting Antibody

The CD8 T cell activation ability dependent on PSMA was compared using bispecific antigen-targeting protein complexes based on the PSMA-specific antibody J591. CD8 T cell activation tests were conducted using PSMA-expressing LNCap cells and PSMA-non-expressing MKN45 cells. The day before, 96-well round plates were coated with an anti-CD3 antibody (Invitrogen, 16-0037-85) at 3 µg/mL, 50 µL per well. The next day, the plates were washed three times with 100 µL of sterile PBS. They were then washed three times with 100 µL of R10 medium (RPMI 1640, 10% FBS, 10 mM Hepes) and blocked with 100 µL of R10 medium at 37 °C for 1 hour. After removing the blocking medium, LNCap and MKN45 cells were counted, adjusted to 7 × 10⁵ cells/mL in R10 medium, and added to the plates at 50 µL per well. Six types of bispecific antigen-targeting protein complexes were prepared at 25 nM and added at 50 µL per well for 30 minutes. Meanwhile, blood was obtained from healthy donors, diluted 1:2 in PBS, and layered over a Leucosep tube (Greiner Bio-One, 227290) containing Picoll (GE Healthcare, 17-1440-12), followed by centrifugation at 1000 g for 30 minutes. The PBMC layer above the Picoll was collected, washed twice by centrifugation at 300 g for 10 minutes in 2% PBS, and counted. CD8-positive T cells were isolated using a CD8 isolation kit (Miltenyi Biotec, 130-096-495). The isolated CD8⁺ T cells were resuspended in R10 medium to 1.4 × 10⁶ cells/mL and added to the plates at 50 µL per well. After 3 days of incubation, the plates were centrifuged at 2000 RPM for 10 minutes, and the supernatants were collected. IFN-γ levels were measured using an IFN-γ ELISA kit (BD, 555142), and Granzyme B levels were measured using a Granzyme B ELISA kit (R&D Systems, DY2906-05), following the respective protocols. CD8 T cell activation was compared based on the measured IFN-γ and Granzyme B levels.

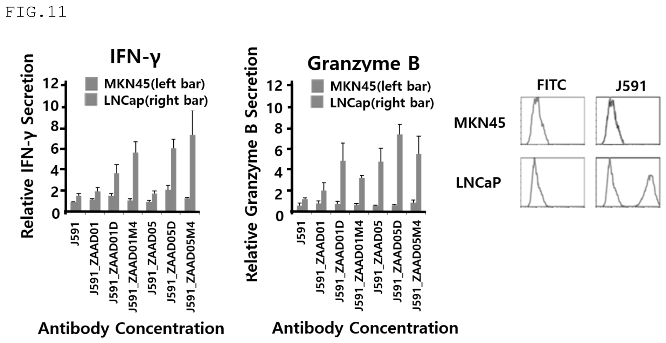

The results are shown in Figure 11.

As shown in Figure 11, the D form and M4 form bispecific antigen-targeting protein complexes exhibited superior CD8 T cell activation ability compared to the basic form. Specifically, J591_ZAAD01D and J591_ZAAD01M4 showed increased levels of secreted IFN-γ and Granzyme B compared to J591_ZAAD01, and J591_ZAAD05D and J591_ZAAD05M4 similarly showed increased secretion of IFN-γ and Granzyme B compared to J591_ZAAD05.

## Claims

1. A bispecific antigen-targeting protein complex comprising:
a first antigen-targeting domain comprising an antibody or an antigen-binding fragment thereof; and
a second antigen-targeting domain comprising affibodies bound to the antibody or the antigen-binding fragment thereof.

2. The bispecific antigen-targeting protein complex according to claim 1, wherein the complex comprises an affibody dimer bound to the C-terminus of the heavy chain of the antibody or the antigen-binding fragment thereof.

3. The bispecific antigen-targeting protein complex according to claim 1, wherein the complex comprises an affibody monomer bound to the C-terminus of each of the heavy chain and the light chain of the antibody or the antigen-binding fragment thereof.

4. The bispecific antigen-targeting protein complex according to claim 1, wherein the complex comprises an affibody dimer bound to the C-terminus of the light chain of the antibody or the antigen-binding fragment thereof.

5. The bispecific antigen-targeting protein complex according to claim 1, wherein the first antigen-targeting moiety targets EGFR (Epidermal Growth Factor Receptor) or PSMA (Prostate-Specific Membrane Antigen), and the second antigen-targeting moiety targets CD137.

6. The bispecific antigen-targeting protein complex according to claim 1, wherein the affibody consists of an amino acid sequence of SEQ ID NO: 1 or SEQ ID NO: 2.

7. The bispecific antigen-targeting protein complex according to claim 1, wherein the antibody or the antigen-binding fragment thereof comprises HCDR1, HCDR2, HCDR3, LCDR1, LCDR2, and LCDR3, each consisting of an amino acid sequence of any one of SEQ ID NOs: 3 to 8; HCDR1, HCDR2, HCDR3, LCDR1, LCDR2, and LCDR3, each consisting of an amino acid sequence of any one of SEQ ID NOs: 27 to 32; or HCDR1, HCDR2, HCDR3, LCDR1, LCDR2, and LCDR3, each consisting of an amino acid sequence of any one of SEQ ID NOs: 51 to 56.

8. The bispecific antigen-targeting protein complex according to claim 1, wherein the antibody or the antigen-binding fragment thereof comprises a heavy chain and a light chain, each consisting of the amino acid sequences of SEQ ID NOs: 9 and 10, respectively; a heavy chain and a light chain, each consisting of the amino acid sequences of SEQ ID NOs: 33 and 34, respectively; or a heavy chain and a light chain, each consisting of the amino acid sequences of SEQ ID NOs: 57 and 58, respectively.

9. The bispecific antigen-targeting protein complex according to claim 1, wherein the bispecific antigen-targeting protein complex comprises an affibody monomer-bound heavy chain consisting of the amino acid sequence of SEQ ID NO: 11 and a light chain consisting of the amino acid sequence of SEQ ID NO: 12; an affibody monomer-bound heavy chain consisting of the amino acid sequence of SEQ ID NO: 19 and a light chain consisting of the amino acid sequence of SEQ ID NO: 20; an affibody monomer-bound heavy chain consisting of the amino acid sequence of SEQ ID NO: 35 and a light chain consisting of the amino acid sequence of SEQ ID NO: 36; an affibody monomer-bound heavy chain consisting of the amino acid sequence of SEQ ID NO: 43 and a light chain consisting of the amino acid sequence of SEQ ID NO: 44; an affibody monomer-bound heavy chain consisting of the amino acid sequence of SEQ ID NO: 59 and a light chain consisting of the amino acid sequence of SEQ ID NO: 60; or an affibody monomer-bound heavy chain consisting of the amino acid sequence of SEQ ID NO: 67 and a light chain consisting of the amino acid sequence of SEQ ID NO: 68.

10. The bispecific antigen-targeting protein complex according to claim 1, wherein the bispecific antigen-targeting protein complex comprises an affibody dimer-bound heavy chain consisting of the amino acid sequence of SEQ ID NO: 13 and a light chain consisting of the amino acid sequence of SEQ ID NO: 14; an affibody dimer-bound heavy chain consisting of the amino acid sequence of SEQ ID NO: 21 and a light chain consisting of the amino acid sequence of SEQ ID NO: 22; an affibody dimer-bound heavy chain consisting of the amino acid sequence of SEQ ID NO: 37 and a light chain consisting of the amino acid sequence of SEQ ID NO: 38; an affibody dimer-bound heavy chain consisting of the amino acid sequence of SEQ ID NO: 45 and a light chain consisting of the amino acid sequence of SEQ ID NO: 46; an affibody dimer-bound heavy chain consisting of the amino acid sequence of SEQ ID NO: 61 and a light chain consisting of the amino acid sequence of SEQ ID NO: 62; or an affibody dimer-bound heavy chain consisting of the amino acid sequence of SEQ ID NO: 69 and a light chain consisting of the amino acid sequence of SEQ ID NO: 70.

11. The bispecific antigen-targeting protein complex according to claim 1, wherein the bispecific antigen-targeting protein complex comprises an affibody monomer-bound heavy chain consisting of the amino acid sequence of SEQ ID NO: 15 and an affibody monomer-bound light chain consisting of the amino acid sequence of SEQ ID NO: 16; an affibody monomer-bound heavy chain consisting of the amino acid sequence of SEQ ID NO: 23 and an affibody monomer-bound light chain consisting of the amino acid sequence of SEQ ID NO: 24; an affibody monomer-bound heavy chain consisting of the amino acid sequence of SEQ ID NO: 39 and an affibody monomer-bound light chain consisting of the amino acid sequence of SEQ ID NO: 40; an affibody monomer-bound heavy chain consisting of the amino acid sequence of SEQ ID NO: 47 and an affibody monomer-bound light chain consisting of the amino acid sequence of SEQ ID NO: 48; an affibody monomer-bound heavy chain consisting of the amino acid sequence of SEQ ID NO: 63 and an affibody monomer-bound light chain consisting of the amino acid sequence of SEQ ID NO: 64; or an affibody monomer-bound heavy chain consisting of the amino acid sequence of SEQ ID NO: 71 and an affibody monomer-bound light chain consisting of the amino acid sequence of SEQ ID NO: 72.

12. The bispecific antigen-targeting protein complex according to claim 1, wherein the bispecific antigen-targeting protein complex comprises a heavy chain consisting of the amino acid sequence of SEQ ID NO: 17 and an affibody dimer-bound light chain consisting of the amino acid sequence of SEQ ID NO: 18; a heavy chain consisting of the amino acid sequence of SEQ ID NO: 25 and an affibody dimer-bound light chain consisting of the amino acid sequence of SEQ ID NO: 26; a heavy chain consisting of the amino acid sequence of SEQ ID NO: 41 and an affibody dimer-bound light chain consisting of the amino acid sequence of SEQ ID NO: 42; a heavy chain consisting of the amino acid sequence of SEQ ID NO: 49 and an affibody dimer-bound light chain consisting of the amino acid sequence of SEQ ID NO: 50; a heavy chain consisting of the amino acid sequence of SEQ ID NO: 65 and an affibody dimer-bound light chain consisting of the amino acid sequence of SEQ ID NO: 66; or a heavy chain consisting of the amino acid sequence of SEQ ID NO: 73 and an affibody dimer-bound light chain consisting of the amino acid sequence of SEQ ID NO: 74.

13. A nucleic acid molecule comprising a nucleotide sequence encoding the bispecific antigen-targeting protein complex according to any one of claims 1 to 12.

14. A recombinant vector comprising the nucleic acid molecule of claim 13.

15. A host cell comprising the recombinant vector of claim 14.

16. A pharmaceutical composition for preventing or treating cancer, comprising a bispecific antigen-targeting protein complex comprising an EGFR (Epidermal Growth Factor Receptor) or PSMA (Prostate-Specific Membrane Antigen) targeting domain comprising an antibody or an antigen-binding fragment thereof, and a CD137 targeting domain comprising affibodies bound to the antibody or the antigen-binding fragment thereof.

17. The pharmaceutical composition according to claim 16, wherein the bispecific antigen-targeting protein complex comprises an affibody dimer bound to the C-terminus of the heavy chain of the antibody or the antigen-binding fragment thereof.

18. The pharmaceutical composition according to claim 16, wherein the bispecific antigen-targeting protein complex comprises an affibody monomer bound to the C-terminus of each of the heavy chain and the light chain of the antibody or the antigen-binding fragment thereof.

19. The pharmaceutical composition according to claim 16, wherein the bispecific antigen-targeting protein complex comprises an affibody dimer bound to the C-terminus of the light chain of the antibody or the antigen-binding fragment thereof.

20. The pharmaceutical composition according to claim 16, wherein the bispecific antigen-targeting protein complex comprises an affibody monomer-bound heavy chain consisting of the amino acid sequence of SEQ ID NO: 11 and a light chain consisting of the amino acid sequence of SEQ ID NO: 12; an affibody monomer-bound heavy chain consisting of the amino acid sequence of SEQ ID NO: 19 and a light chain consisting of the amino acid sequence of SEQ ID NO: 20; an affibody monomer-bound heavy chain consisting of the amino acid sequence of SEQ ID NO: 35 and a light chain consisting of the amino acid sequence of SEQ ID NO: 36; an affibody monomer-bound heavy chain consisting of the amino acid sequence of SEQ ID NO: 43 and a light chain consisting of the amino acid sequence of SEQ ID NO: 44; an affibody monomer-bound heavy chain consisting of the amino acid sequence of SEQ ID NO: 59 and a light chain consisting of the amino acid sequence of SEQ ID NO: 60; or an affibody monomer-bound heavy chain consisting of the amino acid sequence of SEQ ID NO: 67 and a light chain consisting of the amino acid sequence of SEQ ID NO: 68.

21. The pharmaceutical composition according to claim 16, wherein the bispecific antigen-targeting protein complex comprises an affibody dimer-bound heavy chain consisting of the amino acid sequence of SEQ ID NO: 13 and a light chain consisting of the amino acid sequence of SEQ ID NO: 14; an affibody dimer-bound heavy chain consisting of the amino acid sequence of SEQ ID NO: 21 and a light chain consisting of the amino acid sequence of SEQ ID NO: 22; an affibody dimer-bound heavy chain consisting of the amino acid sequence of SEQ ID NO: 37 and a light chain consisting of the amino acid sequence of SEQ ID NO: 38; an affibody dimer-bound heavy chain consisting of the amino acid sequence of SEQ ID NO: 45 and a light chain consisting of the amino acid sequence of SEQ ID NO: 46; an affibody dimer-bound heavy chain consisting of the amino acid sequence of SEQ ID NO: 61 and a light chain consisting of the amino acid sequence of SEQ ID NO: 62; or an affibody dimer-bound heavy chain consisting of the amino acid sequence of SEQ ID NO: 69 and a light chain consisting of the amino acid sequence of SEQ ID NO: 70.

22. The pharmaceutical composition according to claim 16, wherein the bispecific antigen-targeting protein complex comprises an affibody monomer-bound heavy chain consisting of the amino acid sequence of SEQ ID NO: 15 and an affibody monomer-bound light chain consisting of the amino acid sequence of SEQ ID NO: 16; an affibody monomer-bound heavy chain consisting of the amino acid sequence of SEQ ID NO: 23 and an affibody monomer-bound light chain consisting of the amino acid sequence of SEQ ID NO: 24; an affibody monomer-bound heavy chain consisting of the amino acid sequence of SEQ ID NO: 39 and an affibody monomer-bound light chain consisting of the amino acid sequence of SEQ ID NO: 40; an affibody monomer-bound heavy chain consisting of the amino acid sequence of SEQ ID NO: 47 and an affibody monomer-bound light chain consisting of the amino acid sequence of SEQ ID NO: 48; an affibody monomer-bound heavy chain consisting of the amino acid sequence of SEQ ID NO: 63 and an affibody monomer-bound light chain consisting of the amino acid sequence of SEQ ID NO: 64; or an affibody monomer-bound heavy chain consisting of the amino acid sequence of SEQ ID NO: 71 and an affibody monomer-bound light chain consisting of the amino acid sequence of SEQ ID NO: 72.

23. The pharmaceutical composition according to claim 16, wherein the bispecific antigen-targeting protein complex comprises a heavy chain consisting of the amino acid sequence of SEQ ID NO: 17 and an affibody dimer-bound light chain consisting of the amino acid sequence of SEQ ID NO: 18; a heavy chain consisting of the amino acid sequence of SEQ ID NO: 25 and an affibody dimer-bound light chain consisting of the amino acid sequence of SEQ ID NO: 26; a heavy chain consisting of the amino acid sequence of SEQ ID NO: 41 and an affibody dimer-bound light chain consisting of the amino acid sequence of SEQ ID NO: 42; a heavy chain consisting of the amino acid sequence of SEQ ID NO: 49 and an affibody dimer-bound light chain consisting of the amino acid sequence of SEQ ID NO: 50; a heavy chain consisting of the amino acid sequence of SEQ ID NO: 65 and an affibody dimer-bound light chain consisting of the amino acid sequence of SEQ ID NO: 66; or a heavy chain consisting of the amino acid sequence of SEQ ID NO: 73 and an affibody dimer-bound light chain consisting of the amino acid sequence of SEQ ID NO: 74.
